# EUROPEAN PATENT APPLICATION

(11) **EP 4 736 894 A1**
(43) Date of publication of application: **06.05.2026**
(21) Application number: 24210082.4
(22) Date of filing: 31.10.2024
(51) Int. Cl.: A61L 26/00, A61K 38/00

(54) **ANTIMICROBIAL PEPTIDE-FUNCTIONALIZED CROSSLINKABLE COPOLYMER, CROSSLINKED COPOLYMER AND HYDROGEL WITH ANTIBACTERIAL ACTIVITY**

(71) Applicant: Universität Siegen, Körperschaft des öffentlichen Rechts, 57078 Siegen (DE); University Medical Center Amsterdam, 1081 HV Amsterdam (NL)
(72) Inventor: Jonas, Ulrich, 55127 Mainz (DE); Atif, Muhammad, 57076 Siegen (DE); Zaat, Sebastian A.J., 1181 NG AMSTELVEEN (NL); Gizem, Babuccu, 1051 JL Amsterdam (NL)
(74) Representative: Michalski Hüttermann & Partner Patentanwälte mbB

(57) **Abstract**

The present invention relates to a crosslinkable copolymer, the crosslinkable copolymer comprising a hydrophilic monomer, a monomer functionalized with a photocrosslinkable group and a monomer functionalized with an antimicrobial peptide that is covalently bound via at least one of its amino groups. The present invention further relates to a crosslinked copolymer and a hydrogel derived therefrom, as well as a wound dressing comprising one or more of the crosslinkable polymer(s), the crosslinked copolymer or the hydrogel.

## Description

The present invention relates to a crosslinkable copolymer and a crosslinked copolymer that can form a hydrogel, which is usable as wound dressing in the diagnosis and/or treatment of bacterial infections.

Healthcare-Associated Infections (HAIs) arise within medical facilities, where a most severe healthcare-associated infection is wound infection. The presence of bacterial infections in wounds, which are often difficult to detect in an early stage, can significantly impede the healing process. Among the various microorganisms responsible for healthcare-associated infections, *Escherichia coli* (*E. coli*) has been identified as a cause of most severe infections.

Various wound dressings that are usable in the diagnosis or treatment of bacterial infections are known, such as wound dressings based on hydrogels. CN 11135341 A describes an intelligent hydrogel dressing for early warning and controllable treatment of infected wound surfaces. The hydrogel consists of four parts: polyvinyl alcohol which is biocompatible and is often used as wound surface dressing, nanoparticles which can perceive pH changes of wound surfaces and have fluorescence resonance energy transferring effects, macromolecules responding to ultraviolet rays and capable of controllably releasing antibiotics, and upper transformation nanoparticles. CN 114159615 A describes a hydrogel wound dressing that can indicate the pH of the wound and is also responsive to ultrasound for drug delivery. The dressing is made up of a pH-sensitive hydrogel matrix that changes color when there is a change in the pH of the wound. In addition, the hydrogel matrix is loaded with drug nanoparticles that are responsive to ultrasound. When ultrasound is applied to the wound, the drug nanoparticles are released from the hydrogel matrix, resulting in enhanced drug delivery to the wound site. CA 2644432 A1 describes a wound dressing that contains both an anti-inflammatory pain-killing agent and a complex of silver ion and a transitional element of Group IV of the periodic system of elements. The wound dressing can provide effective pain relief and anti-inflammatory benefits while also offering antimicrobial properties due to the presence of silver ions. The transitional element of Group IV of the periodic system of elements is added to enhance the antimicrobial properties of the silver ions.

US 2009209897 A1 describes a photoactivated antimicrobial wound dressing comprising a photocatalytic membrane. The membrane comprises a bacterial cellulose hydrogel membrane with a photosensitizer coated on its surface. When the photosensitizer is activated by light, it produces reactive oxygen species that can kill bacteria on a wound.

Wound dressings for bacterial monitoring comprising sensor elements or electronic devices have been described. US 2021/0000409 A1 describes a sensor system for non-implantable medical devices that can detect and identify microbiological infections. The system uses a combination of sensing technologies to provide real-time detection of pathogens and can be integrated with wireless communication for data transmission. WO 2018/211458 A1 describes a wound covering that allows for wound monitoring and therapeutic agent delivery. The wound covering comprises a sensor element that undergoes a change in appearance in response to a change in a wound, and a therapeutic agent delivery system for delivering therapeutic agents to the wound site. The sensor element comprises fibers comprising a hydrogel and sensor particles. US 10702153 B2 describes a wound dressing that includes reusable electronics for wireless monitoring of the wound. The dressing can monitor various aspects of the wound, such as temperature, moisture, and pH levels. The electronics are attached to the dressing using a snap-fit mechanism, making them easy to attach and remove.

Also, chromogenic substrates for the identification of bacteria are known. WO 2018/060711 A1 discloses a device for detecting the presence of microorganisms in wounds. A genetically engineered microbial sensor, such as Resazurim, 10-acetyl-3,7-dihydroxyphenoxazine or a tetrazolium salt, produces a detectable signal in response to the presence of a target bacterium, which signal can be measured using various detection techniques, such as fluorescence, luminescence, or electrochemical sensing. CN 109439725 A describes a chromogenic medium containing one chromogenic substrate of 4-nitrophenyl-beta-D-glucopyranoside, o-nitrophenol beta-D glucuronide, 4-nitrophenyl-beta-D-glucopyranoside, 4-methylumbelliferyl-beta-D-glucuronide and 5-bromo-4-chloro-3-indoxyl-N-acetyl-beta-D-glucosaminide. The chromogenic substrate interacts with glucosaccharase of *Escherichia coli* to release a chromogenic group. Applications in food testing are described. 5-Bromo-4-chloro-1*H*-indol-3-yl-β-D-glucopyranosiduronic acid (X-Gluc) is commercially available for the colorimetric detection of glucuronidase activity, such as via Carl Roth GmbH & Co. KG.

Chitosan hydrogels functionalized with chromogenic substrates have been described. Mir Morteza Sadat Ebrahimi and Holger Schönherr, dx.doi.org/10.1021/la501482u, Langmuir 2014, 30, 7842-7850, describe chitosan hydrogel-based platform for the detection of enzymes, where the fluorogenic substrate alanyl-alanylphenylalanine-7-amido-4-methylcoumarin (AAP-AMC) was covalently conjugated via amide bond formation to chitosan. Mir Morteza Sadat Ebrahimi et al., ACS Appl. Mater. Interfaces 2015, 7, 20190-20199, report a self-reporting hydrogel for the rapid in situ detection of bacterial enzymes, where chitosan hydrogel films on solid backing supports were equipped with a reporting function for the enzyme β-glucuronidase (β-GUS). Mir Morteza Sadat Ebrahimi et al., European Polymer Journal 81 (2016) 257-265, report chitosan hydrogel films that were equipped with fluorogenic substrates for the detection of a-galactosidase (a-Gal) and b-galactosidase (b-Gal). Mir Morteza Sadat Ebrahimi et al., European Polymer Journal 72 (2015) 180-189, describe a rapid remote detection of Escherichia coli via a reporter-hydrogel coated glass fiber tip, where chitosan was functionalized with the chromogenic substrate 5-bromo-4-chloro-3-indolyl-b-D-glucuronide (XGlcA), which releases a non-leaching dimerized indigo dye into the hydrogel upon reaction with the enzyme b-glucuronidase (b-GUS), which is secreted by >98% of all known Escherichia coli strains. Zhiyuan Jia et al., Bioactive Materials 6 (2021) 4286-4300, describe a multiplexed detection and differentiation of bacterial enzymes and bacteria by color-encoded sensor hydrogels. Zhiyuan Jia et al., Macromol. Symp. 2018, 379, 1600178, describe bacteria detection by enzyme responsive hydrogels where chitosan films were functionalized with the chromogenic substrate 4-nitrophenyl-β-D-glucuronide (PNPG), the chromogenic substrate 5-bromo-4-chloro-3-indolyl-β-D-glucuronide (X-Gluc) and the fluorogenic substrate 4-methylumbelliferyl-β-D-glucuronide (MUG).

Further, a use of antibacterial peptides for the treatment of diseases caused by bacterial infections has been described. Anna de Breij et al., Sci. Transl. Med. 10, eaan4044 (2018) provide a list of LL-37 derived peptides and describe that the antimicrobial peptide SAAP-148 combats drug-resistant bacteria and biofilms. WO 2015/088344 A1 describes antimicrobial peptides comprising an amino acid sequence LKKLYKRLVKILKRWWRYLKRPVR or a variant thereof and used thereof. Riool M et al., Advanced Functional Materials 27, 20 (2017) describe the "Controlled Release of LL-37-Derived Synthetic Antimicrobial and Anti-Biofilm Peptides SAAP-145 and SAAP-276". de Breij A et al., J Control Release 222:1-8, 2016, describe the "Prevention of Staphylococcus aureus biomaterial-associated infections using a polymer-lipid coating containing the antimicrobial peptide OP-145". Riool M et al., BBA Biomembranes 1862, 8, 2020, describe that "Thrombocidin-1-derived antimicrobial peptide TC19 combats superficial multi-drug resistant bacterial wound". Omardien S et al., Biochim Biophys Acta Biomembr 1860:2416-2427, 2018, describe "Synthetic antimicrobial peptides delocalize membrane bound proteins thereby inducing a cell envelope stress response". Kwakman PH et al., Antimicrob Agents Chemother 50:3977-83, 2006, describe "Treatment and prevention of Staphylococcus epidermidis experimental biomaterial-associated infection by bactericidal peptide 2". CN 111892646 A describes antibacterial peptide derivatives, where amino acid residues, except lysine and arginine, in the antibacterial peptides GF-17 and SAAP-148 are replaced with lysine. The structure of the antibacterial peptides is modified to increase their antibacterial activity and reduce their toxicity. WO 2022/226297 A1 describes antimicrobial lipopeptides comprising a peptide which is a truncation of the amino acid sequence KRIXQRIKDFLR, wherein X is W or V, and a fatty acid that is conjugated to the N-terminus of the peptide.

An overview of antimicrobial peptides is described by Andrea Giuliani et al., CEJB 2(1) 2007 1-33. Wound healing activity of the human antimicrobial peptide LL37 are described in the bachelor thesis of Megan Dion "Wound Healing Effects of a Modified Collagen-Binding Antimicrobial Peptide LL37", December 2015 and Reinaldo Ramo et al., Peptides 32 (2011) 1469-1476. Matthias Gabriel et al. describe "Preparation of LL-37-Grafted Titanium Surfaces with Bactericidal Activity", Bioconjugate Chem. 2006, 17, 548-550.

Moniek Schmitz (2022) "Antimicrobial Supramolecular Biomaterials: From Molecular Design to Screening" Phd Thesis 1 (Research TU/e / Graduation TU/e), Biomedical Engineering, Eindhoven University of Technology, reports a prevention of experimental biomaterial-associated infection via an antimicrobial peptide SAAP-148 functionalized supramolecular coating on titanium.

WO 2017/212345 A2 describes a chemical entity comprising an anchor, an enzyme recognition site and an indicator region for the detection of microbial infections in wounds. The technology relates to detection of bacterial and/or viral substances, such as enzymes and substrates, at the wound sites.

EP 2958933 B1 describes a hydrogel comprising a plurality of amphiphilic peptides and/or peptoids capable of self-assembling into three-dimensional macromolecular nanofibrous networks, which entrap water and form said hydrogel, wherein at least a portion of said plurality of amphiphilic peptides and/or peptoids is chemically, e.g. covalently, cross-linked.

Hydrogels with various cross-linking structures are described. WO 2024/094645 A1 describes hydrophilic (co)polymers having chemically reactively (covalently) bound anchor groups and/or crosslinker molecules that permit attachment to the parent substrate or linkage of these polymers to networks.

A. Brunsen et al., J. Mater. Chem., 2012, 22, 19590, describe photocrosslinkable dextran hydrogel films as substrates for osteoblast and endothelial cell growth. Silvia Freese et al., Gels 2020, 6, 1, describe copolymer networks covalently bonded to flexible polyethylene (PE) sheets. The syntheses of acrylates and methacrylates of the surfactants Triton X-100, Brij 35, and Ecosurf EH-3 or EH-9 and their incorporation into copolymers with acrylamide (PAM) and N-(4-benzoylphenyl)acrylamide are reported. Further, photocrosslinked polymer networks were described to be prepared from the copolymers on corona-treated polyethylene sheets, which can be swollen with aqueous solution to form hydrogel layers.

S. Atefyekta et al., "Antibiofilm elastin-like polypeptide coatings: functionality, stability, and selectivity", Acta Biomaterialia 83 (2019) 245-256, describe an antimicrobial peptide, RRPRPRPRPWWWW-NH₂ (RRP9W4N), that was used to modify elastin-like polypeptide (ELP) surface coatings containing cell-adhesive peptide domains (RGD) using covalent chemistry. S. Atefyekta et al. in "Antimicrobial peptide-functionalized mesoporous hydrogels", ACS Biomaterials Science & Engineering 7(4) (2021) 1693-1702, describe an antimicrobial peptide RRPRPRPRPWWWW-NH₂ (RRP9W4N), that is covalently attached to amphiphilic and ordered mesoporous Pluronic F127 hydrogels. The AMP-hydrogels showed high antibacterial activity against Staphylococcus epidermidis, Staphylococcus aureus, Pseudomonas aeruginosa, methicillin-resistant S. aureus (MRSA), and multidrug-resistant Escherichia coli for up to 24 h.

Still, bacterial infections in wounds, particularly skin wounds, provide a serious burden on the healthcare system.

The object of the present invention is therefore to provide a means allowing treatment of bacterial infections in wounds.

The object is achieved by the crosslinkable copolymer according to claim 1 and the crosslinked copolymer according to claim 10. The object further is achieved by the hydrogel according to claim 11. The object further is achieved by the wound dressing according to claim 12. The object further is achieved by the hydrogel or the wound-dressing for use according to claims 13 and 14. Advantageous embodiments are the subject of the dependent claims. The embodiments may be combined freely unless the context clearly indicates otherwise.

Accordingly, a crosslinkable copolymer is provided, the crosslinkable copolymer comprising a hydrophilic monomer, a monomer functionalized with a photocrosslinkable group, and a monomer functionalized with an antimicrobial peptide that is covalently bound via at least one of its amino groups.

A copolymer with a covalently bound antimicrobial peptide is provided that allows crosslinking and forming of a hydrogel of the crosslinked copolymer, which copolymer and hydrogel are usable in a wound dressing and allow antimicrobial treatment of a wound. Surprisingly it was found that the chemically immobilized antimicrobial peptide in the polymer matrix nearly fully retained its antimicrobial activity.

It was found that the copolymer was non-toxic to human lung fibroblasts. Photocrosslinking of the photocrosslinkable group by UV light yielded a polymer network that forms a hydrogel after swelling with aqueous medium. Both, a synthesized copolymer comprising covalently bound antimicrobial peptide SAAP-148 in solution and photocrosslinked hydrogels derived therefrom showed good antimicrobial activity against different strains of *Escherichia coli, Staphylococcus aureus, Pseudomonas aeruginosa,* and *Acinetobacter baumannii.* The covalent attachment of the antimicrobial peptide prevents leaching from the polymer matrix, and the hydrogel was found to be able to retain its antimicrobial activity for 14 days in phosphate-buffered saline and for up to 48 hours in human blood plasma. This provides a considerable lifetime of the functionality of the antimicrobial peptides. The copolymer and hydrogel provide a potential approach for antimicrobial wound dressing applications, suitable as alternative to antibiotics and other drugs. This provides an improvement in infection management of wounds.

In embodiments, the crosslinkable copolymer comprises a monomer functionalized with a chromogenic dye that is convertible, such as cleavable, by a bacterial enzyme. Advantageously, such embodiments can address the dual challenge of timely detection and efficient elimination of *E*. *coli* and other microorganisms responsible for wound infections.

A chromogenic dye that is converted specifically by a bacterial enzyme or protein allows the detection of such bacteria, e.g. in a wound. Including a monomer containing a chromogenic dye indicative of a bacterial enzyme provides therapeutic and diagnostic functionalities that can be chemically immobilized in one copolymer. This allows to merge both diagnostic and therapeutic capabilities usable in a single medical device.

The term "chromogenic dye" generally refers to a colorless compound comprising or consisting of a chromophore, i.e. a chemical group that, after enzymatic conversion, usually cleavage, provides in a product with a distinctive color, and a specific enzymatic substrate. Chromogenic dyes are synthetically produced and are designed to possess a selectivity similar to the natural substrate for the respective enzyme. Each chromogenic dye has a corresponding enzyme, which catalyzes the separation of the chromophore group, resulting in a colored chromophore product that confirms the existence and activity of the enzyme. The color can be qualitatively or quantitatively assessed as a readout for the actual bacterial enzyme. A copolymer or hydrogel comprising a chromogenic dye thus are useful for enzymatic detection of bacterial contamination of a wound.

In embodiments, the chromogenic dye is selected from the group comprising 4-nitrophenyl-β-D-glucuronide (PNPG), 5-bromo-4-chloro-3-indolyl-β-D-glucuronide (X-Gluc), 4-methylumbelliferyl-β-D-glucuronide (MUG) and 4-methylumbelliferyl-α-D-glucuronide (MUD). Chemically, X-Gluc is the cyclohexyl ammonium salt of 5-bromo-4-chloro-3-indolyl-β-D-glucuronic acid. The corresponding enzyme for X-Gluc is beta-glucuronidase. The chromophore product that is released upon enzymatic cleavage is chloro-bromoindigo, which provides an indigo blue color. Spectrophotometrically, its intensity can be detected at 615 nm. Also, for 4-nitrophenyl-β-D-glucuronide (PNPG) the corresponding enzyme is beta-glucuronidase. The chromophore product that is released upon enzymatic cleavage provides a yellow color.

Beta-glucuronidase (β-GUS) is secreted by more than 98% of the known Escherichia coli (E. coli) strains. When a copolymer comprising a monomer functionalized with 5-bromo-4-chloro-3-indolyl-β-D-glucuronide (X-Gluc) or a hydrogel formed thereof will come in contact with bacterial beta-glucuronidase, for example in an infected wound, the β-linked indole derivative of X-GLUC can be cleaved by β-GUS and is released into the hydrogel, where the liberated molecule can dimerize to form an indigo derivative, which can be simply detected by naked eye. In a similar way, when a copolymer comprising a monomer functionalized with 4-nitrophenyl β-D-glucuronide (PNPG) or a hydrogel formed thereof will come in contact with bacterial beta-glucuronidase, for example in an infected wound, the PNPG chromogenic substrate is also cleaved by β-GUS enzyme and liberates 4-nitrophenol (4-NP) from the glucuronide, whose yellow color, which is due to the deprotonation of 4-NP, can be observed by naked eye.

In embodiments, the monomer functionalized with the chromogenic dye is N-(6-aminohexyl)acrylamide. In other embodiments, the monomer functionalized with the chromogenic dye may be N-(6-aminohexyl)methacrylamide.

The chromogenic dye may be covalently bound to the monomer directly or via an intermediary or linker group, such as an alkylene diamine, preferably hexamethylene diamine. The monomer can be modified with N-tert-butyloxycarbonyloxy (Boc)-protected hexamethylene diamine by using post-polymerization modification (PPM) chemistry. To immobilize the chromogenic dye in a polymer backbone, a usable synthetic route can provide that the chromogenic dye and a monomer can be modified to contain an active ester and free amine group respectively. The chromogenic dye, for example, 4-nitrophenyl-β-D-glucuronide (PNPG) or 5-bromo-4-chloro-3-indolyl β-D-glucuronide (X-GLUC) can be modified, for example, with carbonyl diimidazole (CDI) as an active ester. The monomer, preferably already being part of a copolymer, such as synthesized by free radical polymerization, can be modified with Boc-protected hexamethylene diamine by using post-polymerization modification (PPM) chemistry. After polymerization, deprotection of the hexamethylene diamine group provides free amines in polymer backbone. The CDI-modified chromogenic dye can be covalently bound to the copolymer via active ester chemistry as known to the skilled person.

The chromogenic dye can be activated with an ester group selected from the group of compounds comprising carbonyldiimidazole (CDI), N-hydroxysuccinimide (NHS), 1-ethyl-3-(3-dimethylaminopropyl)carbodiimide (EDC), N, N'-disuccinimidyl carbonate (DSC), benzotriazol-1-yl-oxytripyrrolidinophosphonium-hexafluorophosphate (PyBOP) and 1-hydroxybenzotriazole (HOBt).

In embodiments, the crosslinkable copolymer comprises a monomer functionalized with a surfactant. A surfactant-modified monomer, such as acrylate, may provide for hydrophilic and hydrophobic balance and add amphiphilicity to the polymer. A surfactant may enhance the adhesion of the crosslinkable copolymer or a crosslinked polymer to other components, such as plastics. For example, wound dressing may contain several layers, including protective outer layers that may be made from polyurethane which is breathable, waterproof and bacteria resistant. The surfactant may also be able to interact with bacterial membranes, although it is not assumed that the surfactant will interrupt bacterial membranes. The copolymer preferably will contain the surfactant in low amounts.

The surfactant may be selected from the group comprising ethylene oxide-propylene oxide copolymer mono(2-ethylhexyl) ethers, poly(oxyethylene) lauryl ethers and 4-(1,1,3,3-tetramethylbutyl)phenyl-polyethylene glycols. In embodiments, the surfactant is an ethylene oxide-propylene oxide copolymer mono(2-ethylhexyl) ether. Commercially available ethylene oxide-propylene oxide copolymer mono(2-ethylhexyl) ethers are ECOSURF^{™} EH-3 and ECOSURF^{™} EH-9. A commercially available poly(oxyethylene) lauryl ether is Brij^{®}35. A commercially available 4-(1,1,3,3-tetramethylbutyl)phenyl-polyethylene glycole is Triton^{®} X-100.

The monomer may be selected from the group comprising acrylate and methacrylate. A reactive form of the monomer can be reacted with the surfactant to yield a monomer functionalized with a surfactant. For example, acryloyl chloride can be reacted with commercially available ECOSURF^{™} EH-3 to yield ECOSURF^{™} EH-3 acrylate monomer.

In embodiments, the surfactant is selected from the group comprising ethylene oxide-propylene oxide copolymer mono(2-ethylhexyl) ethers, poly(oxyethylene) lauryl ethers and 4-(1,1,3,3-tetramethylbutyl)phenyl-polyethylene glycoles and the monomer is selected from the group comprising acrylate and methacrylate. Preferred monomers functionalized with a surfactant are selected from the group comprising EH3 acrylate, EH9 acrylate, poly(oxyethylene) lauryl ether (Brij 35) acrylate (B35A), poly(oxyethylene) lauryl ether (Brij 35) methacrylate (B35M), 4-(1,1,3,3-tetramethylbutyl)phenyl-polyethylene glycole (Triton X-100) acrylate and 4-(1,1,3,3-tetramethylbutyl)phenyl-polyethylene glycole (Triton X-100) methacrylate.

The copolymer comprises an antimicrobial peptide that is covalently bound to the monomer via at least one of its amino groups. The antimicrobial peptide preferably is derived from or inspired by natural cationic antimicrobial protein, such as from LL-37. The cathelicidin anti-microbial peptide denoted LL-37 is the active form of the human cathelicidin antimicrobial peptide (CAMP). Other preferred antimicrobial peptides may be selected from thrombocidin-derived peptides, such as derived from the thrombocidin 1 and 2 family. Other preferred antimicrobial peptides may be selected from peptides derived from the bactericidal/permeability-increasing protein (BPI). The peptide will contain a functional, i.e. antimicrobially active, peptide motif, and may have a length of about 10 to 30 amino acids. The peptide preferably has a length of about 15 to 25 amino acids. Such peptides will provide antimicrobial activity but will not interfere with the structure of the copolymer and derived hydrogel.

In embodiments, the antimicrobial peptide is selected from the group of LL-37-derived peptides comprising LKKLWKRVFRIWKRIFRYLKRPVR (P139, SEQ ID NO: 8), LRRLWKRLURIIKRIYRQLKRPVR (P140, SEQ ID NO: 9), LRRLYKRVFRLLKRWWRYLKRPVR (P141, SEQ ID NO: 10), LRRLWKRLVKILKRWFRYLRRPVR (P142, SEQ ID NO: 11), LRRLYKRVVKLWKRLFRQLRRPVR (P143, SEQ ID NO: 12), LKKLYKRVAKIWKRWIRYLKKPVR (P144, SEQ ID NO: 13), LKRLYKRLAKLIKRLYRYLKKPVR (SAAP-145) (SEQ ID NO: 2) LKKLYKRLFKILKRILRYLRKPVR (P146, SEQ ID NO: 14), LKKLWKRLARLLKRFIRQLRRPVR (P147, SEQ ID NO: 15), LKRVWKRVFKLLKRYWRQLKKPVR (SAAP-148) (SEQ ID NO: 1), LKKVYKRLARLLKRYIRYLRRPVR (P149, SEQ ID NO: 16), LKKVWKRVARLIKRWFRYLRRPVR (P150, SEQ ID NO: 17), LKKLYKRLFKLWKRLYRYLKKPVR (P151, SEQ ID NO: 18), LRRVYKRLARLIKRYLRQLKKPVR (P152, SEQ ID NO: 19), LRKLWKRVVKIWKRYLRQLRRPVR (P153, SEQ ID NO: 20), LRKLWKRLAKIIKRLYRYLRRPVR (P154, SEQ ID NO: 21), LKKVYKRVARLIKRLFRYLKRPVR (P155, SEQ ID NO: 22), LRRLWKRLVKLWKRFFRYLKKPVR (P156, SEQ ID NO: 23), LKKVWKRVFRILKRFLRYLKRPVR (P157, SEQ ID NO: 24), LRRVYKRLFRLWKRIIRQLRRPVR (P158, SEQ ID NO: 25), LKRLYKRVFRLLKRYYRQLRRPVR (P159 or SAAP-159, SEQ ID NO: 26), LKKLWKRLARLWKRIIRQLKKPVR (P160, SEQ ID NO: 27), LRRVWKRVARIIKRLYRYLKRPVR (P161, SEQ ID NO: 28), LKRLWKRLFKILKRYYRYLRRPVR (P162, SEQ ID NO: 29), LRRLWKRVFKIIKRLFRQLKKPVR (P163, SEQ ID NO: 30) and LKRVWKAVFKLLKRYWRQLKKPVR (SAAP-276) (SEQ ID NO: 3).

In embodiments, the antimicrobial peptide is selected from the group of peptides comprising LKRVWKRVFKLLKRYWRQLKKPVR (SAAP-148) (SEQ ID NO: 1), LKRLYKRLAKLIKRLYRYLKKPVR (SAAP-145) (SEQ ID NO: 2), LKRVWKAVFKLLKRYWRQLKKPVR (SAAP-276) (SEQ ID NO: 3), IGKEFKRIVERIKRFLRELVRPLR (OP-145) (SEQ ID NO: 4), LRCMCIKWWSGKHPK (TC19) (SEQ ID NO: 5), LRAMCIKWWSGKHPK (TC84) (SEQ ID NO: 6), and GKWKLFKKAFKKFLKILAC (BP2) (SEQ ID NO: 7).

The antimicrobial peptide may comprise acetyl groups, amide groups or acetyl groups and amide groups. In embodiments, the N-terminal amino acid comprises an acetyl group, the C-terminal amino acid comprises an amide group or N-terminal and C-terminal amino acid comprise an acetyl group and an amide group, respectively. In embodiments, the antimicrobial peptide is selected from the group of peptides comprising acetyl-LKRVWKRVFKLLKRYWRQLKKPVR (SAAP-148) (SEQ ID NO: 1), acetyl-LKRLYKRLAKLIKRLYRYLKKPVR-amide (SAAP-145) (SEQ ID NO: 2), acetyl-LKRVWKAVFKLLKRYWRQLKKPVR-amide (SAAP-276) (SEQ ID NO: 3), acetyl-IGKEFKRIVERIKRFLRELURPLR-amide (OP-145) (SEQ ID NO: 4), LRCMCIKWWSGKHPK-amide (TC19) (SEQ ID NO: 5), LRAMCIKWWSGKHPK-amide (TC84) (SEQ ID NO: 6) and GKWKLFKKAFKKFLKILAC (BP2) (SEQ ID NO: 7). In embodiments, the antimicrobial peptide is an LL-37-derived peptide selected from the group of SAAP-148, SAAP-145, SAAP-276 and OP-145. The peptide may be selected from SAAP-148, SAAP-145 and SAAP-276. In preferred embodiments, the antimicrobial peptide is SAAP-148. In other embodiments, the antimicrobial peptide is a peptide derived from the thrombocidin-1 family, optionally selected from TC19 and TC84. In other embodiments, the antimicrobial peptide may be BP2.

The copolymer comprises a hydrophilic monomer, a monomer functionalized with a photocrosslinkable group, and a monomer functionalized with an antimicrobial peptide. The copolymer further may comprise a monomer functionalized with a chromogenic dye and/or a monomer functionalized with a surfactant. The copolymer may be an acrylate or acrylate/methacrylate copolymer or comprise a majority of acrylate and/or methacrylate monomers.

The copolymer contains a hydrophilic monomer that in embodiments forms a biocompatible main monomer. A use of hydrophilic properties in polymers is particularly advantageous in applications where water and oxygen transmission is desirable without sacrificing basic mechanical or physical properties of the polymer backbone. Such properties are particularly advantageous for medical uses, such as for a use in wound dressings.

In embodiments, the hydrophilic monomer is selected from the group comprising hydroxyethyl acrylamide (HEAAm), hydroxypropyl acrylamide (HPAm), N-vinyl-2-pyrrolidone (NVP), N,N-dimethylacrylamide (DMAA), methacrylamide (MAA), 2-hydroxyethyl methacrylate (HEMA), 2-hydroxypropyl methacrylate (HPMA), 2-methyl-2-oxazoline, 2-ethyl-2-oxazoline, 2-n-propyl-2-oxazoline and 2-isopropyl-2-oxazoline. In embodiments, the hydrophilic monomer is based on acrylate and/or methacrylate. In embodiments, the hydrophilic monomer is selected from hydroxyethyl acrylamide (HEAAm), hydroxypropyl acrylamide (HPAm), N-vinyl-2-pyrrolidone (NVP), N,N-dimethylacrylamide (DMAA), methacrylamide (MAA), 2-hydroxyethyl methacrylate (HEMA) and 2-hydroxypropyl methacrylate (HPMA) monomers. In embodiments, the hydrophilic monomer is hydroxyethyl acrylamide (HEAAm). HEAAm as the main component of the copolymer provides advantageous hydrophilic and biocompatible properties. An additional benefit of using HEAAm is its antifouling property.

In embodiments, the hydrophilic monomer provides the main component of the copolymer. In embodiments, the monomers that are functionalized are selected from acrylate monomers and methacrylate monomers.

The crosslinkable copolymer further comprises a monomer functionalized with a photocrosslinkable group. In embodiments, the monomer is selected from the group comprising acrylamide and methacrylamide. In embodiments, the photocrosslinkable group is selected from the group comprising benzophenone, (4-benzoylphenyl)methyl]dimethyl[3- (prop-2-enamido)propyl]azanium chloride (BPQAAm), 4-(2,3-epoxypropyloxy) benzophenone (EBP), bis(2,4,6-trimethylbenzoyl)phenylphosphine oxide (BAPO) and 2,2-dimethoxy-2-phenylacetophenone (DMPA). In embodiments, the photocrosslinkable group is benzophenone. The benzophenone group can be crosslinked under the exposure of UV without photo-oxidative damage to the antimicrobial peptide. In embodiments, the monomer functionalized with a photocrosslinkable group is selected from (4-benzoylphenyl)acrylamide and 4-benzoylphenyl)methacrylamide.

The copolymer comprises a monomer functionalized with an antimicrobial peptide. The antimicrobial peptide is covalently bound to the monomer via at least one of its amino groups. In embodiments, the antimicrobial peptide is covalently bound to the monomer via its N-terminal amino group. The covalent binding requires a reactive amino group in the peptide that forms a covalent bond with a functional group of the monomer. Reactivity and binding can be supported chemically, such as by "active ester" chemistry as known to the skilled person and described in Chem. Rev. 2016, 116, 1434-1495 and European Polymer Journal 41 (2005) 1569-1575.

In embodiments, the antimicrobial peptide binds to a functionalized acrylate or methacrylate monomer. The acrylate or methacrylate monomer can be functionalized with a pentafluorophenole group to support binding of the antimicrobial peptide. In embodiments of the crosslinkable copolymer, the antimicrobial peptide binds to an acrylate monomer functionalized with pentafluorophenole via reaction of at least one of its amino group(s) forming an amide bond with the acrylate. In embodiments, the antimicrobial peptide binds to an acrylate monomer functionalized with pentafluorophenole via reaction of its N-terminal amino group forming an amide bond with the acrylate.

In the preparation of the copolymers described herein, an unspecified distribution of the repeating monomer units is assumed. A systematic source-based nomenclature for copolymers identifying the constituent monomers and providing a description of the sequence arrangement of the monomeric units is achieved by giving the formula (or citing the names of the constituent monomers after the prefix "poly"), and by placing between the formulae or names of each pair of monomers the italicized connective "-CO-" to denote the unspecified arrangement by which the two monomeric units are related in the structure.

In the polymer structures, the number in the index corresponds to the molar ratio of the feed-in. For example, x in a range of 0.6 to 0.95, y in a range of 0.01 to 0.03, z in a range of 0.01 to 0.10, m in a range of 0.0001 to 0.1, and n in a range of 0.01 to 0.03, means that 0.6 to 0.95 mol% of the hydrophilic monomer, 0.01 to 0.03 mol% of the monomer functionalized with a photocrosslinkable group, 0.01 to 0.10 mol% of the monomer functionalized with a chromogenic dye, 0.0001 to 0.1 mol% of the monomer functionalized with the covalently bound antimicrobial peptide and 0.01 to 0.03 mol% of the monomer functionalized with a surfactant were used as the molar ratio at the beginning of the polymerization.

The crosslinkable polymer may have the following general formula (I): 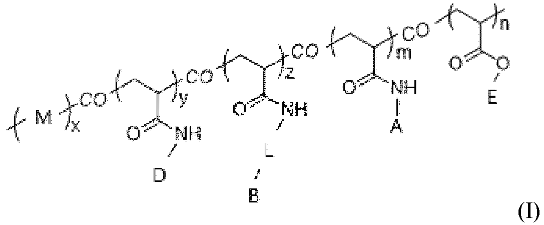 wherein:
- M: is a hydrophilic monomer, optionally selected from the group of monomers comprising hydroxyethyl acrylamide (HEAAm), hydroxypropyl acrylamide (HPAm), N-vinyl-2-pyrrolidone (NVP), N,N-dimethylacrylamide (DMAA), methacrylamide (MAA), 2-hydroxyethyl methacrylate (HEMA), 2-hydroxypropyl methacrylate (HPMA), 2-methyl-2-oxazoline, 2-ethyl-2-oxazoline, 2-n-propyl-2-oxazoline, and 2-isopropyl-2-oxazoline.
- A: is an antimicrobial peptide, optionally derived from or inspired by LL-37, or derived from thrombocidin 1 or 2 or BPI, optionally selected from the group of P139-P144, SAAP-145, P146, P147, SAAP-148, P149-P163, SAAP-276, OP-145, TC19, TC84 and BP2, optionally selected from the group of SAAP-148, SAAP-145, SAAP-276, OP-145, TC19, TC84 and BP2,
- B: is a chromogenic dye convertible, optionally cleavable, by a bacterial enzyme, optionally selected from the group comprising 4-nitrophenyl β-D-glucuronide (PNPG), 5-bromo-4-chloro-3-indolyl-β-D-glucuronide (X-Gluc), 4-methylumbelliferyl β-D-glucuronide (MUG) and 4-methylumbelliferyl α-D-glucuronide (MUD),
- D: is a photocrosslinkable group, optionally selected from the group comprising benzophenone, (4-benzoylphenyl)methyl]dimethyl[3-(prop-2-enamido)propyl]azanium chloride (BPQAAm), 4-(2,3-epoxypropyloxy) benzophenone (EBP), bis(2,4,6-trimethylbenzoyl)phenylphosphine oxide (BAPO) and 2,2-dimethoxy-2-phenylacetophenone (DMPA),
- E: is a surfactant, optionally selected from the group of ethylene oxide-propylene oxide copolymer mono(2-ethylhexyl) ethers, poly(oxyethylene) lauryl ethers and 4-(1,1,3,3-tetramethylbutyl)phenyl-polyethylene glycols,
- L: is a linker group, optionally an alkylene amine, preferably hexamethylene amine,
- x: is in a range of 0.6 to 0.95, preferably 0.88,
- y: is in a range of 0.01 to 0.03, preferably 0.02,
- z: is in a range of 0 to 0.10, preferably in a range of 0.01 to 0.10, more preferably 0.07,
- m: is in a range of 0.0001 to 0.1, preferably 0.001, and
- n: is in a range of 0 to 0.03, preferably in a range of 0.01 to 0.03, more preferably 0.02.

For the description of the hydrophilic monomer, antimicrobial peptide, chromogenic dye, photocrosslinkable group, surfactant and linker further reference is made to the description above. The crosslinkable copolymer optionally can comprise a monomer comprising a chromogenic dye convertible cleavable by a bacterial enzyme and/or a monomer comprising a surfactant. Thus, the indices z and/or n may be 0.

The hydrophilic monomer may be selected from hydroxyethyl acrylamide (HEAAm) and hydroxypropyl acrylamide (HPAm), and optionally may be hydroxyethyl acrylamide (HEAAm). The photocrosslinkable group may be benzophenone.

In embodiments, the crosslinkable polymer has the following formula (1): 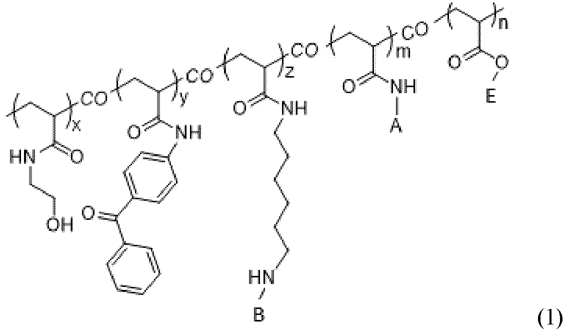 wherein:
- A: is an antimicrobial peptide selected from the group of SAAP-148 (SEQ ID NO: 1), SAAP-145 (SEQ ID NO: 2), SAAP-276 (SEQ ID NO: 3), OP-145 (SEQ ID NO: 4), TC19 (SEQ ID NO: 5), TC84 (SEQ ID NO: 6) and BP2 (SEQ ID NO: 7),
- B: is a chromogenic dye selected from the group comprising 4-nitrophenyl β-D-glucuronide (PNPG), 5-bromo-4-chloro-3-indolyl-β-D-glucuronide (X-Gluc), 4-methylumbelliferyl β-D-glucuronide (MUG) and 4-methylumbelliferyl α-D-glucuronide (MUD),
- E: is a surfactant selected from the group of ethylene oxide-propylene oxide copolymer mono(2-ethylhexyl) ethers, poly(oxyethylene) lauryl ethers and 4-(1,1,3,3-tetramethylbutyl)phenyl-polyethylene glycols,
- x: is in a range of 0.6 to 0.95, preferably 0.88,
- y: is in a range of 0.01 to 0.03, preferably 0.02,
- z: is in a range of 0 to 0.10, preferably in a range of 0.01 to 0.10, more preferably 0.07,
- m: is in a range of 0.0001 to 0.1, preferably 0.001,
- n: is in a range of 0 to 0.03, preferably in a range of 0.01 to 0.03, more preferably 0.02.

For the description of the antimicrobial peptide, chromogenic dye and surfactant further reference is made to the description above.

In embodiments, the antimicrobial peptide is selected from the group of peptides comprising LKRVWKRVFKLLKRYWRQLKKPVR (SAAP-148) (SEQ ID NO: 1), LKRLYKRLAKLIKRLYRYLKKPVR (SAAP-145) (SEQ ID NO: 2), LKRVWKAVFKLLKRYWRQLKKPVR (SAAP-276) (SEQ ID NO: 3), IGKEFKRIVERIKRFLRELVRPLR (OP-145) (SEQ ID NO: 4), LRCMCIKWWSGKHPK (TC19) (SEQ ID NO: 5), LRAMCIKWWSGKHPK (TC84) (SEQ ID NO: 6), and GKWKLFKKAFKKFLKILAC (BP2) (SEQ ID NO: 7).

In embodiments, the antimicrobial peptide is selected from the group of acetyl-LKRVWKRVFKLLKRYWRQLKKPVR (SAAP-148, SEQ ID NO: 1), acetyl-LKRLYKRLAKLIKRLYRYLKKPVR-amide (SAAP-145, SEQ ID NO: 2), acetyl-LKRVWKAVFKLLKRYWRQLKKPVR-amide (SAAP-276, SEQ ID NO: 3), acetyl-IGKEFKRIVERIKRFLRELVRPLR-amide (OP-145, SEQ ID NO: 4), LRCMCIKWWSGKHPK-amide (TC19, SEQ ID NO: 5), LRAMCIKWWSGKHPK-amide (TC84, SEQ ID NO: 6) and GKWKLFKKAFKKFLKILAC (BP2, SEQ ID NO: 7). In preferred embodiments, the antimicrobial peptide is SAAP-148.

In embodiments, the chromogenic dye is selected from 4-nitrophenyl β-D-glucuronide (PNPG) and 5-bromo-4-chloro-3-indolyl-β-D-glucuronide (X-Gluc). In embodiments, the surfactant is an ethylene oxide-propylene oxide copolymer mono(2-ethylhexyl) ether, preferably ECOSURF^{™} EH-3 and ECOSURF^{™} EH-9.

In embodiments, the crosslinkable copolymer has the following formula (2):

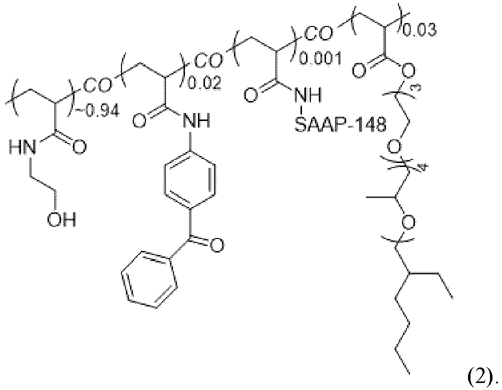

The copolymer can be produced by synthesizing poly[(hydroxy ethyl acrylamide)-*co*-(4-benzophenone acrylamide)-*co*-(pentafluorophenyl acrylate)-*co*-(ECOSURF EH-3 acrylate)], in short poly(HEAAm-*co*-BPAAm-*co*-PFPA-co-EH3A), by free radical polymerization and subsequent modification of the active ester groups with the amine groups of the antimicrobial peptide SAAP-148, resulting in the copolymer. It was found that the copolymer was not cytotoxic to human lung fibroblasts.

In embodiments, the crosslinkable copolymer has the following formula (3): 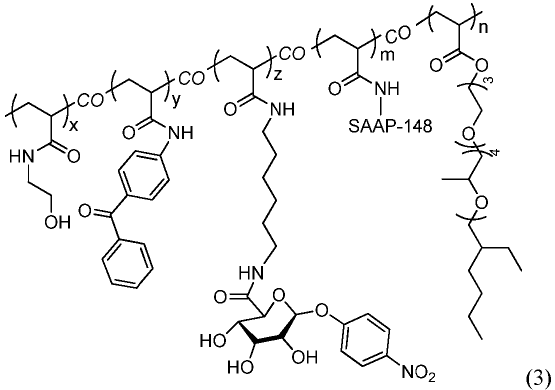 wherein x is in a range of 0.6 to 0.95, preferably 0.88,
- y: is in a range of 0.01 to 0.03, preferably 0.02,
- z: is in a range of 0.01 to 0.10, preferably 0.07,
- m: is in a range of 0.0001 to 0.1, preferably 0.001, and
- n: is in a range of 0.01 to 0.03, preferably 0.02.

It was found that the copolymer was not cytotoxic to human lung fibroblasts. The copolymer of formula (3) can be produced by synthesizing poly[(hydroxy ethyl acrylamide)-*co*-(4-benzophenone acrylamide)-*co*-(pentafluorophenyl acrylate)-*co*-(ECOSURF EH-3 acrylate)] and modifying with, for example 10 mol% of, 4-nitrophenyl-β-D-glucuronide (PNPG) modified with protected, such as Boc-protected, hexamethylene diamine and, for example 0.1 mol% of, the antimicrobial peptide SAAP-148, resulting in the copolymer poly[(hydroxy ethyl acrylamide)-*co*-(4-benzophenone acrylamide)-*co-*(4-nitrophenyl-β-D-glucuronide)-*co*-(SAAP-148 acrylamide)-*co*-(ECOSURF EH-3 acrylate)] of formula (3).

Alternatively, the copolymer can be synthesized by a) modifying poly[(hydroxy ethyl acrylamide)-*co-*(4-benzophenone acrylamide)-*co*-(pentafluorophenyl acrylate)-*co*-(ECOSURF EH-3 acrylate)] with SAAP-148 by using click chemistry, b) modifying poly[(hydroxy ethyl acrylamide)-*co*-(4-benzophenone acrylamide)-*co*-(pentafluorophenyl acrylate)-*co*-(ECOSURF EH-3 acrylate)] with Boc-protected hexamethylene diamine by using post-polymerization modification (PPM) chemistry and reacting with active ester (such as CDI)-activated PNPG, and c) mixing the resulting products of a) and b).

In embodiments, the crosslinkable copolymer has the following formula (4): 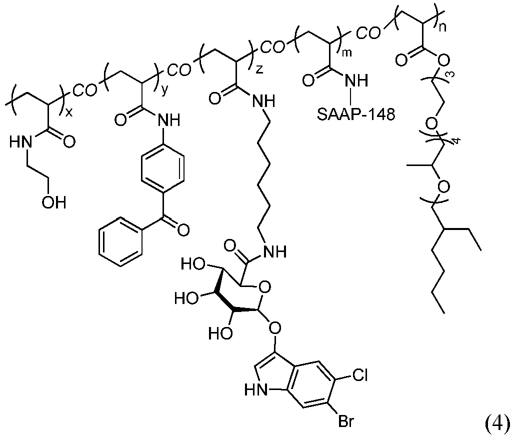 wherein
- x: is in a range of 0.6 to 0.95, preferably 0.88,
- y: is in a range of 0.01 to 0.03, preferably 0.02,
- z: is in a range of 0.01 to 0.10, preferably 0.07,
- m: is in a range of 0.0001 to 0.1, preferably 0.001, and
- n: is in a range of 0.01 to 0.03, preferably 0.02.

It was found that the copolymer was not cytotoxic to human lung fibroblasts. The copolymer of formula (4) can be produced by synthesizing poly[(hydroxy ethyl acrylamide)-*co*-(4-benzophenone acrylamide)-*co*-(pentafluorophenyl acrylate)-*co*-(ECOSURF EH-3 acrylate)] and modifying with, for example 10 mol% of, 5-bromo-4-chloro-3-indolyl β-D-glucuronide (X-GLUC) modified with protected, such as Boc-protected, hexamethylene diamine and, for example 0.1 mol% of, the antimicrobial peptide SAAP-148, resulting in the copolymer poly[(hydroxy ethyl acrylamide)-*co*-(4-benzophenone acrylamide)-*co*-(5-bromo-4-chloro-3-indolyl β-D-glucuronide)-*co*-(SAAP-148 acrylamide)-*co*-(ECOSURF EH-3 acrylate)] of formula (4).

Alternatively, the copolymer of formula (4) can be synthesized by a) modifying poly[(hydroxy ethyl acrylamide)-*co*-(4-benzophenone acrylamide)-*co*-(pentafluorophenyl acrylate)-*co*-(ECOSURF EH-3 acrylate)] with SAAP-148 by using click chemistry, b) modifying poly[(hydroxy ethyl acrylamide)-*co-*(4-benzophenone acrylamide)-*co*-(pentafluorophenyl acrylate)-*co*-(ECOSURF EH-3 acrylate)] with Boc-protected hexamethylene diamine by using post-polymerization modification (PPM) chemistry and reacting with active ester (such as CDI)-activated X-GLUC, and c) mixing the resulting products of a) and b).

A further aspect refers to a crosslinked copolymer, obtained by photo-induced crosslinking of one or more of the crosslinkable polymer(s) according to the invention. For the description of the crosslinkable copolymer, hydrophilic monomer, antimicrobial peptide, chromogenic dye, photocrosslinkable group, surfactant and linker reference is made to the description above.

The crosslinkable copolymer according to the invention comprises photocrosslinkable groups. They may be crosslinked by methods known to a skilled person. The photocrosslinkable groups particularly may be photocrosslinkable with UV radiation. It has been found that the benzophenone group can be crosslinked under the exposure of UV without photo-oxidative damage to the antimicrobial peptide.

Thus, a crosslinked copolymer is obtainable by photo-induced crosslinking of at least the crosslinkable copolymer comprising a biocompatible monomer, a photocrosslinkable monomer and a monomer comprising the antimicrobial peptide covalently bound via its N-terminal amino group. The crosslinkable copolymer according to the invention particularly may be a photocrosslinkable copolymer and the crosslinked copolymer particularly may be a photocrosslinked copolymer.

The crosslinkable copolymer optionally can comprise a surfactant, a chromogenic dye or a surfactant and a chromogenic dye. A crosslinked copolymer comprising a biocompatible monomer, a photocrosslinkable monomer and a monomer comprising the antimicrobial peptide covalently bound via its N-terminal amino group and a dye and optionally a surfactant may be produced by reacting a crosslinkable copolymer comprising a biocompatible monomer, a photocrosslinkable monomer and a monomer comprising an ester group and optionally a surfactant with the antimicrobial peptide and the chromogenic dye. Alternatively, such a crosslinked copolymer is obtainable by crosslinking a first crosslinkable copolymer comprising a biocompatible monomer, a photocrosslinkable monomer and a monomer comprising an antimicrobial peptide covalently bound via its N-terminal amino group to an ester group and a second crosslinkable copolymer comprising a biocompatible monomer, a photocrosslinkable monomer and a monomer comprising a chromogenic dye, optionally either the first, the second or both photocrosslinkable monomers may comprise a surfactant.

A further aspect refers to a hydrogel comprising the crosslinked copolymer according to the invention. Photocrosslinking of the photocrosslinkable group such as the benzophenone units by UV light will yield a polymer network that can form a hydrogel after swelling with aqueous medium. A hydrogel is obtainable from a crosslinked copolymer that is synthetically available. This allows full control during the synthesis of the copolymer and over the components of the hydrogel. Also, stability of diagnostic and treatment ability of the hydrogel as well as toxicity behavior has been confirmed. The results provide opportunities for autonomous identification and effective treatment of bacterial strains in wound infections.

A hydrogel can be obtained by swelling the crosslinked copolymer with water, aqueous buffers, but also with water-containing fluid, such as wound secretion or pus, seeping from a wound. Both the synthesized SAAP-148-modified polymer in solution and the photocrosslinked hydrogels showed good antimicrobial activity against strains of Escherichia coli, Staphylococcus aureus, Pseudomonas aeruginosa, and Acinetobacter baumannii, including multidrug-resistant strains, frequently found in wound infections.

The photocrosslinkable copolymer described herein covalently immobilizes the antimicrobial peptides and can enhance the long-term functionality of the antimicrobial peptides, ensuring that the covalently immobilized peptides retain their activity in biological environment. Upon processing, a resulting hydrogel can exhibit a sustained and effective antimicrobial activity against various bacterial species without the need for additional antibiotics. It was found that the hydrogel was not cytotoxic and retained its antimicrobial activity under the tested conditions. Such non-cytotoxic and durable copolymer-based hydrogel provides a promising agent for antimicrobial wound dressing applications, offering an effective alternative to traditional antibiotics.

Embodiments of a hydrogel derived from a copolymer further containing a chromogenic dye cleavable by bacterial enzymes as described herein were found to allow detection and be effective against the tested bacterial strains.

A further aspect refers to a wound dressing, comprising one or more of the crosslinkable polymer(s) according to the invention, the crosslinked copolymer according to the invention or the hydrogel according to the invention.

The term "wound dressing" as used herein refers to materials applied to wounds to promote healing, protect the wound from infection, or prevent further injury. Wound dressing may have different forms and types, depending on the respective purpose and benefit. One of the primary functions of wound dressings is to provide a moist environment for wound healing, which promotes the growth of healthy cells and facilitates the process of healing.

The crosslinked copolymer provided on a wound dressing will swell upon contact with water in a wound and form a hydrogel. Also, the crosslinkable polymer(s) according to the invention may be provided on a wound dressing, such as in solution form. Such soluble polymers may advantageously function as precursors to the insoluble crosslinked networks.

A wound dressing comprising a copolymer or hydrogel functionalized with an antimicrobial peptide as described allows antimicrobial treatment of a wound. Particulary a wound dressing a copolymer or hydrogel functionalized with an antimicrobial peptide and functionalized with a chromogenic dye provides the ability to diagnose and signal the presence of bacteria at an early stage and to autonomously induce an on-demand treatment.

Particularly hydrogel dressings can create a moist wound environment conducive to wound healing. Hydrogel dressings are a common choice for dry wounds with minimal drainage, as they can help prevent desiccation, promote autolysis, and reduce wound pain. Hydrogel dressings may be used to hydrate dry or dehydrated wounds, for burn wounds or for necrotic wounds.

A further aspect refers to the hydrogel as described herein or the wound dressing as described herein for use in the diagnosis and/or treatment of a bacterial infection, particularly of a wound. Again, for the description of the hydrogel, the crosslinked copolymer, the crosslinkable copolymer, hydrophilic monomer, antimicrobial peptide, chromogenic dye, photocrosslinkable group, surfactant and linker reference is made to the description above.

In embodiments, the bacterial infection is caused by *Escherichia coli (E. coli), Staphylococcus aureus*, particularly *Methicillin Resistant Staphylococcus aureus* (MRSA), *Pseudomonas aeruginosa (P. aeruginosa)* and/or *Acinetobacter baumanii (A. baumanii).* Both, the synthesized SAAP-148-modified polymer in solution and the photocrosslinked hydrogels have been shown to provide good antimicrobial activity against strains of *Escherichia coli*, *Staphylococcus aureus*, *Pseudomonas aeruginosa*, and *Acinetobacter baumannii,* including multidrug-resistant strains, frequently found in wound infections.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:
- Figure 1: Comparison of ¹H NMR of poly(HEAAm-co-BPAAm-co-PFPA-co-EH3A) and poly(HEAAm-co-BPAAm-co-SAAP-148-co-EH3A).
- Figure 2: Analysis of antimicrobial activity and stability assessment of covalently bound SAAP-148 in a hydrogel.
- Figure 3: Number of CFU of *E. coli* DH5α per well counted for PBS used for rinsing a hydrogel with covalently bound SAAP-148 and pure PBS
- Figure 4: Results of the modified Miles Misra analysis of the antimicrobial properties of single chain polymer poly(HEAAm-*co*-SAAP-148) in solution against different planktonic bacterial strains.
- Figure 5: Results of the modified Miles Misra analysis for 2 hours incubation time, where "A" illustrates the result for PBS, "B" for the polymer PHEAAm without antimicrobial peptide SAAP-148, and "C" for the peptide-modified poly(HEAAm-*co*-SAAP-148).
- Figure 6: Analysis of the antimicrobial activity against E. coli DH5α for SAAP-148-modified hydrogels of poly(HEAAm-*co*-SAAP-148) in contact with human blood plasma.
- Figure 7: Assessment of metabolic activity by the WST-1 assay for NHLF cells in contact with solutions of single chain polymers without (PHEAAm) and with covalently attached antimicrobial peptides (poly(HEAAm-co-SAAP-148)).
- Figure 8: Color changes of copolymers poly(HEAAm-*co*-PNPG) and poly(HEAAm-*co*-X-GLUC) functionalized with chromogenic dye in solutions by enzymatic reaction of different strains of E. coli, S. aureus, P. aeruginosa, and A. baumannii.

### Materials

Reagents and chemicals were obtained from commercial sources and used as received unless otherwise noted. N-(2- hydroxyethyl) acrylamide (97%), pentafluorophenyl (98%), and laboratory grade ECOSURF EH-3 (abbreviated here as EH3) were purchased from Sigma Aldrich. Azobisisobutyronitrile (AIBN) was recrystallized from methanol (98.5%; VWR Chemicals). Azobisisobutyronitrile was recrystallized from methanol. Triethyl amine (99.5%, Sigma Aldrich) was distilled before use. Hexamethylene diamine (97%, Sigma Aldrich), carbonyldiimidazole (97%, Alfa Aesar), were used. Trifluoroacetic acid was purchased from Carl Roth in PEPTIPURE^{®} ≥99,9% quality. 4-nitrophenyl-β-D-glucuronide acid (PNPG) (99%) and 5-bromo-4-chloro-3-indolyl β-D-glucuronide (X-GLUC) (99%) were purchased from Sigma Aldrich. β-glucuronidase purified from E. coli (β-GUS, 1 000 000-5 000 000 units/g protein, E.C.3.2.1.31; type IX-A, lyophilized powder) and β-glucuronidase (β-GUS), isolated from *E. coli* (1000-5000 units/mg protein, E.C.3.2.1.31; Type IX-A) were purchased from Sigma-Aldrich. Triethyl amine (97%, Sigma Aldrich) was distilled prior to use. Roswell Park Memorial Institute (RPMI) medium supplemented with 20 mM Hepes and L-glutamine, without sodium bicarbonate (further referred to as RPMI) was bought from Sigma Aldrich. Sodium dodecyl sulfate (SDS) was purchased from Sigma-Aldrich. Fetal bovine serum (FBS) and Dulbecco's Modified Eagle's Medium (DMEM) were obtained from Thermo Fisher Scientific. Normal human lung fibroblasts (NHLF) were acquired from Lonza, and pooled human plasma (from four donors) was sourced from Sanquin, Amsterdam, The Netherlands. SAAP-148 were bought from Bisca biochemical. Deionized water (mili-Q) was used throughout the experiments.

### Methods

### Nuclear magnetic resonance (NMR):

¹H NMR (400 MHz) and ¹³C NMR (100 MHz) spectra were recorded on a Bruker AV 400 spectrometer. The spectra were acquired at 25 ± 1 °C using deuterium chloroform (CDCl3), deuterium Oxide (D2O), and deuterium dimethyl sulfoxide-d6 (DMSO-d6) as solvents while tetramethylsilane (TMS) was selected as an internal standard. 1DSpectra were analysed with MestreNova 9.

### Bacteria culture and growth:

Prior to experiments, clinical and laboratory strains of *Escherichia coli* (PC 1568, DH5α, DC 10B, B12 C1, TOP 10, PC 2348AR4, ATCC 8739, and ML35 (ATCC 43827), *Staphylococcus aureus* strain JAR060131, a standard laboratory strain of *Pseudomonas aeruginosa* PAO1, and a multidrug-resistant strain of *Acinetobacter baumannii* RUH875 from frozen stocks were grown at 37 °C on blood agar plates for 16-18 h. From each plate, overnight cultures were prepared in lysogeny broth (Oxoid) for all *E. coli* strains, and in tryptic soy broth (Oxoid) for *S*. *aureus*, *P. aeruginosa*, and *A. baumannii*, and incubated at 37 °C for 18-24 h at 120 rpm. Prior to each experiment, bacteria were cultured to the mid-logarithmic growth phase in their respective media. Bacteria were washed twice with phosphate-buffered saline (PBS; pH=7.0) and diluted in RPMI to 1 × 10⁶ colony forming units (CFU)/mL based on the optical density at 620 nm.

### Example 1: Polymer synthesis

### 1.1: Synthesis of ECOSURF^{™} EH3 acrylate (EH3A) surfactant monomer

EH3 acrylate was synthesized following the procedure given in S. Freese et al., Gels 2020, 6, 1.

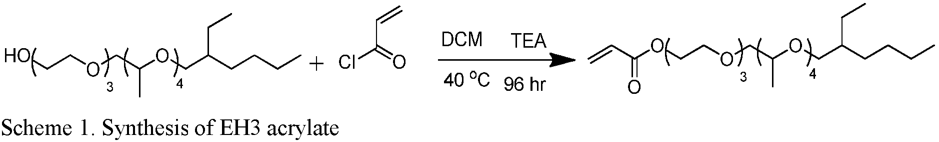

EH3 (ECOSURF^{™} EH3, 9.61 ml, 18.76 mmol) was dissolved in dichloromethane (20 mL) in a two necked flask with a magnetic stirring bar inside. Triethylamine (TEA) in dichloromethane (20 mL) was added and the reaction mixture was stirred for one hour in an ice bath. Afterwards a solution of prop-2-enoyl chloride in dichloromethane (40 mL) was added under argon dropwise with strong stirring. The reaction mixture was purged with argon, sealed, and stirred under the reflux condenser for 96 hours. To purify the reaction, the mixture was first neutralized with aqueous sodium hydroxide (0.1 M, 3 mL), and the solvent was removed. The residue was taken in an aqueous sodium hydroxide (1 M, 100 mL) and the aqueous phase was washed with dichloromethane (2x, 50 mL). In the next step the organic phases were combined, dried with magnesium sulphate and the solvent was evaporated. The residue was dissolved in dichloromethane (100 mL) and washed with aqueous hydrochloride solution (2x, 1 M, 50 mL). The aqueous phase was extracted with dichloromethane (50 mL) and the organic phases were combined, dried with magnesium sulphate and the solvent was evaporated. The slightly yellow viscous liquid was dried in a vacuum overnight. Successful synthesis was confirmed via¹H NMR.

### 1.2: Synthesis of 4-benzophenone acrylamide (BPAAm) photo crosslinker monomer

### 4-Benzophenone acrylamide was synthesized as described in B. Peng, et al., ACS Applied Materials & Interfaces 12(51) (2020) 56753-56766

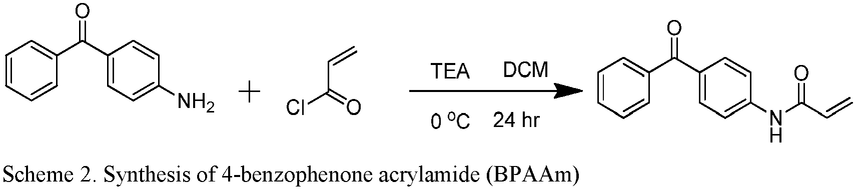

4-amino benzophenone (1.97 g, 10.0 mmol) was dissolved in dichloromethane (80 mL) containing Potassium carbonate (1.66 g, 12.0 mmol) in a two necked flash with a magnetic stirring bar inside. Prop-2-enoyl chloride (0.98 mL, 12.0 mmol) was dissolved in dichloromethane (50 mL) and added to a dropping funnel. The solution was added dropwise to the reaction mixtures, which was cooled with ice under vigorous stirring. After 48 hours, the product was cleaned with column chromatography (silica gel, CH₂Cl₂: EtOAc = 8:1). The product was separated by the side product by using a TLC (silica gel, CH₂Cl₂: EtOAc = 8:1). Afterwards the slightly brownish solid was dried with vacuum overnight. Successful synthesis was confirmed via ¹H NMR.

### 1.3: Synthesis of pentafluorophenyl acrylate (PFPA) as an active ester monomer

Pentafluorophenyl acrylate (PFPA) was synthesized according to the procedure given in P. Theato, Journal of Polymer Science Part A: Polymer Chemistry 46(20) (2008) 6677-6687.

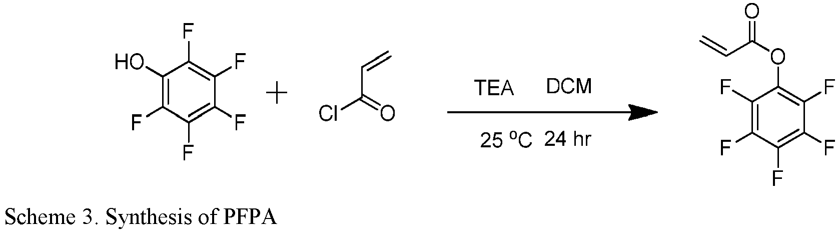

Pentafluorophenyl (10.0 g, 54.3 mmol) was dissolved in dichloromethane (~ 90 mL) and cooled to 0 °C. Triethylamine (8 mL, 5.8 g, 1 mol eq.) was added and a mixture of acryloyl chloride (5 mL, 5.6 g) in dichloromethane (15 mL) was slowly added dropwise. Another 100 mL of DCM was added, and the mixture was stirred overnight at room temperature (~ 20 °C). The mixture was extracted with brine (3x, ~ 150 mL), dried over sodium sulphate, the solvent was removed under reduced pressure and the product was finally dried under vacuum (< 1 mbar) and obtained colorless liquid. Successful synthesis was confirmed via ¹H NMR.

### 1.4: Synthesis of poly(HEAAm-co-BPAAm-co-PFPA-co-EH3A) and modification with antimicrobial peptide SAAP-148 to yield poly(HEAAm-co-BPAAm-co-SAAP-148-co-EH3A)

In a first step, a copolymer was synthesized containing HEAAm as main monomer, BPAAm as photocrosslinker monomer, ECOSURF^{™} EH3A as surfactant monomer, and PFPA as an active ester monomer to enable post polymerization modification (PPM). The resultant copolymer (poly(HEAAm-co-BPAAm-co-PFPA-*co*-EH3A)) was modified with antimicrobial peptide SAAP-148 by using active ester chemistry to form poly(HEAAm-co-BPAAm-co-SAAP-148-co-EH3A) according to the synthesis protocol of scheme 4:

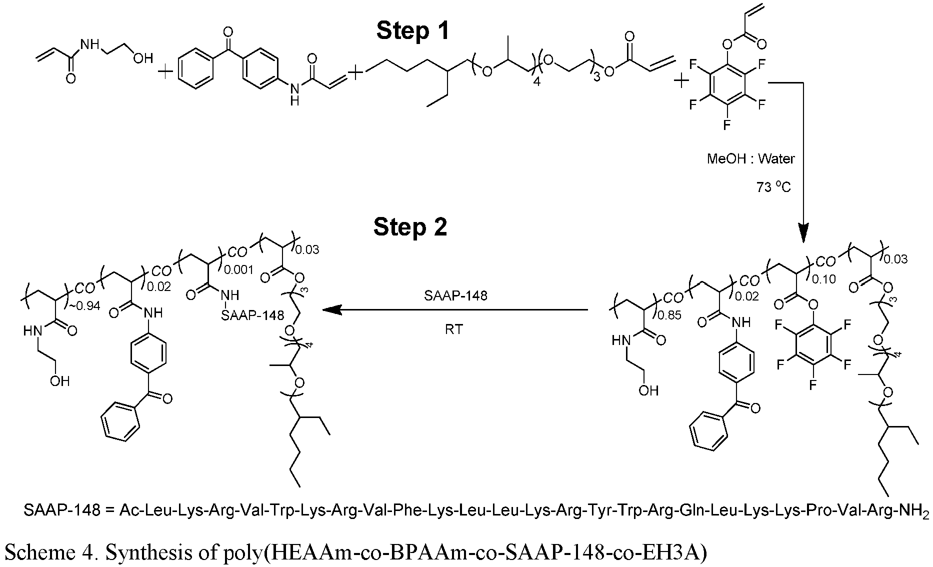

According to Step 1, hydroxyethyl acrylamide (HEAAm) (500 mg, 4.41 mmol), EH3A (72.60 mg, 0.13 mmol), BPAAm (22.20 mg, 0.08 mmol), PFPA (73.60 mg, 0.30 mmol), and azobisisobutyronitrile (AIBN) (7.25 mg, 0.44 mmol, 1 mol %) were transferred to a schlenk tube with a magnetic stirring bar. The mixture was filled with 10 mL of methanol, purged with argon for 30 minutes, and heated at 65 °C for 24 hours. After precipitation in diethyl ether and overnight vacuum drying, the white product (abbreviated poly(HEAAm-*co*-BPAAm-*co*-PFPA-*co*-EH3A)) was obtained. ¹H NMR, ¹⁹F NMR, and FTIR corroborated a successful synthesis.

According to Step 2, 100 milligrams (0.42 mmol) of poly(HEAAm-*co*-BPAAm-*co*-PFPA-*co*-EH3A) were dissolved in 5 milliliter of an 80:20 water-dioxane solvent mixture. To this solution, 0.88 mL of SAAP-148 (1 mg/mL) and 100 µL of triethylamine were added. The reaction was carried out at 40°C for 48 hours. After this period, 75 µL of ethanolamine (10 equivalents) was added to the reaction mixture and the reaction was continued for an additional 24 hours at room temperature. Upon completion, the reaction mixture was freeze dried. Subsequently, the freeze-dried product was subjected to the dialysis for 24 hours. After purification (dialysis), the product was freeze-dried to yield a final polymer (denoted poly(HEAAm-*co*-BPAAm-*co*-SAAP-148-*co*-EH3A). The obtained products were confirmed by ¹H NMR and ¹⁹F NMR.

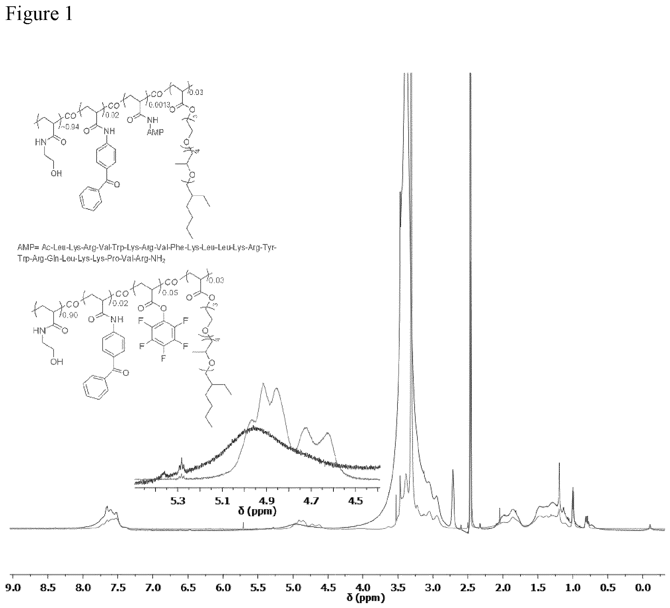

Figure 1 shows a comparison of the ¹H NMR spectra of poly(HEAAm-co-BPAAm-co-PFPA-co-EH3A) and poly(HEAAm-co-BPAAm-co-SAAP-148-co-EH3A). As can be taken from figure 1, the ¹H NMR spectrum of the copolymer modified with antimicrobial peptide (AMP) showed additional resonances at 2.72 ppm, 2.80-3.82 ppm, and 4.63-5.31 ppm compared to the spectrum of the copolymer before modification, which can be attributed to the immobilized SAAP-148 within the polymer backbone. Further, ¹⁹F NMR spectra lacked any signal compared to the pre-modified polymer, signifying the complete consumption of active ester groups.

This confirms that a crosslinkable copolymer poly(HEAAm-*co*-BPAAm-*co*-SAAP-148-*co*-EH3A) comprising a hydrophilic monomer (HEAAm), a monomer functionalized with a photocrosslinkable group (BPAAm) and a monomer functionalized with a covalently bound antimicrobial peptide (SAAP-148) was synthesized.

In the following, the copolymer poly[(hydroxy ethyl acrylamide)-*co*-(4-benzophenone acrylamide)-*co-*(SAAP-148 acrylamide)-*co*-(ECOSURF EH3 acrylate)] is denoted poly(HEAAm-*co*-BPAAm-*co-*SAAP-148-*co*-EH3A) or in a shortened form as "poly(HEAAm-*co*-SAAP-148)" or "PHEAAm-*co-*SAAP-148". This abbreviation also refers to the crosslinked copolymer, where the text differentiates when referring to the crosslinkable or the crosslinked copolymer.

Further, the SAAP-148-free copolymer poly[(hydroxy ethyl acrylamide)-*co*-(4-benzophenone acrylamide)-*co*-(ECOSURF EH3 acrylate)] was used as a control and is denoted poly(HEAAm-*co-*BPAAm-*co*-EH3A) or in a shortened form as "poly(HEAAm)" or "PHEAAm". This abbreviation also refers to the crosslinked copolymer, where the text differentiates when referring to the crosslinkable or the crosslinked copolymer.

### Example 2: Photocrosslinking and hydrogel formation

A solution of 25 mg/mL of the respective polymer in methanol and water (90:10; v/v) was prepared in a petri dish and dried over a hotplate at 40 °C overnight. The dried polymer layer was irradiated inside UV photocrosslinker (UVP ultraviolet crosslinker CL-1000) at a wavelength of Z=365 nm (1 h=13.0 J/cm²) for 30 min to form the crosslinked polymer network structure.

The photocrosslinked polymer network structure was swollen in water to form a hydrogel. The resulting hydrogel immobilized in the 96-well plate was then employed for microbiological experiments to investigate the stability of covalently immobilized antimicrobial peptide and the antimicrobial properties of the hydrogel modified with antimicrobial peptide in human blood plasma.

### Example 3: Stability test of covalently immobilized antimicrobial peptide in the hydrogel

A phosphate-buffered saline (PBS) washing test was performed for the duration of 2 weeks to assess the long-term stability of covalently immobilized antimicrobial peptide in a photocrosslinked polymer network structure, which after swelling with water forms the hydrogel, and compare it with the physical absorption of AMP in the hydrogel.

In this test, antimicrobial peptide-modified hydrogel of poly(HEAAm-*co*-SAAP-148) and antimicrobial peptide-free hydrogel of poly(HEAAm) (PHEAAm) as a control were covalently deposited in the wells of a 96-well plate. For physical absorption of antimicrobial peptide, the poly(HEAAm) hydrogel was incubated with a 10 µM solution of SAAP-148 in water. Afterwards, 100 µL of PBS (pH = 7) was added to both types of hydrogels, and samples were incubated at 37 °C for up to 14 days. At the end of each time point (1 hour, 1 day, 7 days, and 14 days) all the liquid was extracted from the wells and then the hydrogels were rinsed three times with PBS. Afterwards, a bacterial suspension of *E. coli* DH5α (10⁶ CFU/mL) in RPMI media was added and incubated overnight. Afterwards, 10 µL aliquots from each well were cultured in a series of increasing dilutions onto the blood agar plates and overnight incubated. Finally, the number of colonies were counted to determine how long each hydrogel type could retain its antibacterial properties.

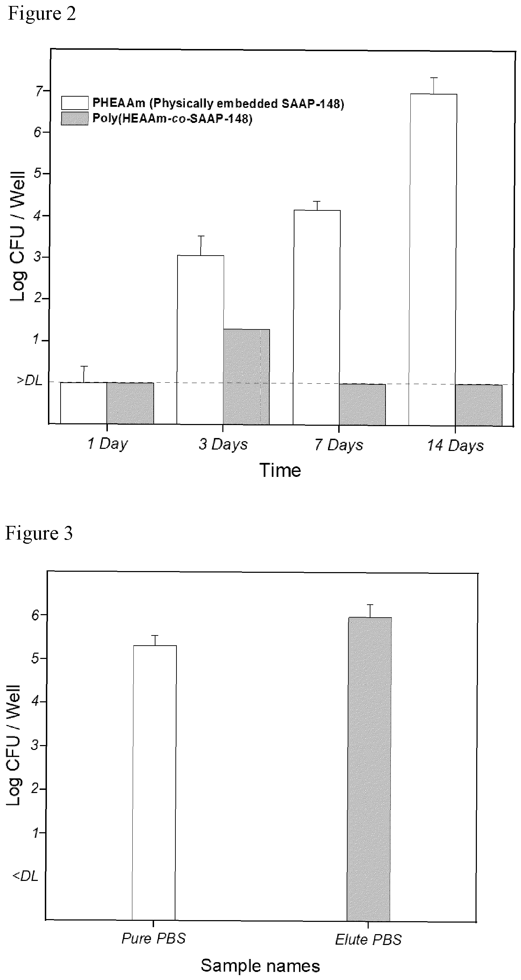

Figure 2 illustrates the results of the analysis of antimicrobial activity and stability assessment of chemically immobilized SAAP-148 in the hydrogel. As can be seen in figure 2, the hydrogel with covalently bound SAAP-148 could retain its antibacterial activity against *E. coli* DH5α for up to 14 days, while the hydrogel containing physically embedded SAAP-148 lost its antimicrobial activity after 3 days. These results confirm the presence of covalently immobilized SAAP-148, as the SAAP-148-modified hydrogels retain their antibacterial activity for an extended period of time while no SAAP-148 was leaching from the hydrogel matrix into the surrounding environment even after 14 days of PBS exposure.

Moreover, after 7 days, the PBS aliquots used for rinsing the hydrogel (with chemically immobilized SAAP-148) were mixed with 10⁶ CFU/mL of *E. coli* DH5α suspension and incubated overnight. From this stock suspension, a dilution series was prepared and 10 µL aliquots were placed onto the blood agar plates, incubated overnight, and then the number of colonies were counted. Pure PBS was taken as control.

Figure 3 provides the respective number of CFU of *E. coli* DH5α per well counted for the PBS used for rinsing the hydrogel and pure PBS. As seen in figure 3, an almost similar number of CFU/well were observed in both samples, indicating that no antimicrobial peptide leached out from the hydrogel matrix into the buffer.

### Example 4: Qualitative analysis of antimicrobial properties of antimicrobial peptide-modified single-chain polymers in solution

Inhibition zone test was performed to analyze the antimicrobial properties of antimicrobial peptide-modified polymer in solution against *E*. *coli, S. aureus, P. aeruginosa, and A. baumannii.*

For the inhibition zone test, 100 µL of bacterial suspensions (10⁶ CFU/mL) of E. coli, S. aureus, P. aeruginosa, and A. baumannii was spread onto tryptone soy agar (TSA) plates, covering the entire agar surface to ensure confluent growth. The plates were left for several minutes to dry. Subsequently, 10 µL aliquots of a solution of antimicrobial peptide-modified copolymer poly(HEAAm-*co*-SAAP-148) and of SAAP-148-free polymer poly(HEAAm-co-BPAAm-co-EH3A) were deposited onto marked locations of the agar surface. PBS was taken as negative control and 120 µM of SAAP-148 solution was taken as positive control. The plates were then incubated overnight. During the next day, pictures were taken to qualitatively analyse the antimicrobial properties of the antimicrobial peptide-modified polymer solution against the bacterial strains by the appearance of the inhibition zone.

No inhibition zone was observed for PBS and for antimicrobial peptide-free polymer poly(HEAAm-co-BPAAm-co-EH3A) in aqueous solution. In contrast, a solution of SAAP-148-modified polymer poly(HEAAm-*co*-SAAP-148 in water caused clearly visible inhibition zones against all the tested bacterial strains. This result demonstrates a high effectiveness of the antimicrobial peptide-modified polymers against the tested bacterial strains by contact killing, with SAAP-148 retaining its activity when covalently immobilized in the polymer backbone.

### Example 5: Quantitative analysis of antimicrobial properties of antimicrobial peptide-modified single-chain polymers in solution

A quantitative analysis of antimicrobial properties of SAAP-148-modified polymer poly(HEAAm-*co-*SAAP-148) in solution was performed with the Miles Misra method against eight strains of *Escherichia coli* (PC 1568, DH5α, DC 10B, B12 C1, TOP 10, PC 2348AR4, ATCC 8739, and ML35 (ATCC 43827)), one strain of *Staphylococcus aureus* (JAR060131), one strain of *Pseudomonas aeruginosa* (01), and one strain of *Acinetobacter baumannii* (RUH875).

100 µL of polymer solutions were added to wells of a 96-well plate (number of replicates n=2), followed by the addition of 100 µL of bacterial suspension (10⁶ CFU/mL in RPMI). The plates were then incubated overnight at 37°C. Afterwards, 10 µL aliquots of each suspension were series-cultured onto blood agar plates with increasing dilution and incubated overnight followed by counting the number of colonies. To determine the impact of SAAP-148 modification, the antimicrobial activity of SAAP-148-modified polymer in solution was compared against both unmodified SAAP-148-free polymer solution of poly(HEAAm) (PHEAAm) and a control of phosphate-buffered saline (PBS).

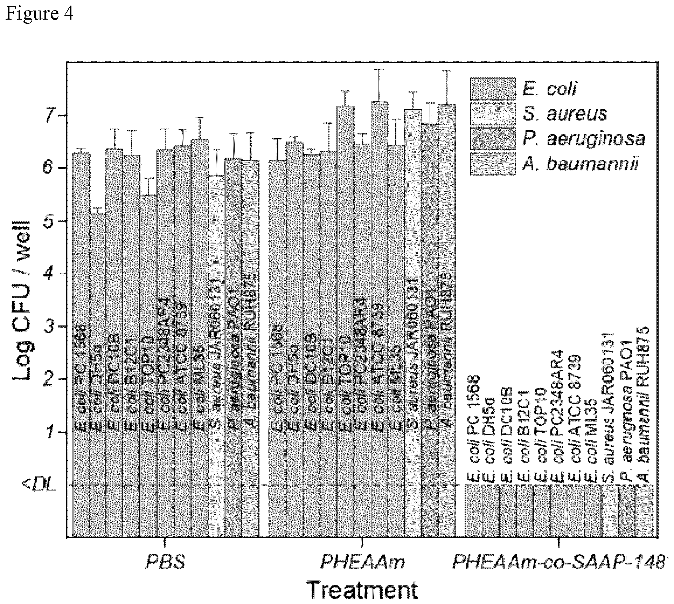

Figure 4 illustrates the results of the modified Miles Misra analysis of the antimicrobial properties of single chain polymer poly(HEAAm-*co*-SAAP-148) in solution against different planktonic bacterial strains. As can be taken from figure 4, a high number of colony forming units (CFU) between 10⁵ and 10⁷ CFU/well were observed for the reference samples without SAAP-148 (PHEAAm) and PBS, which indicates no detrimental effect on the bacterial growth of the tested strains. On the other hand, a clear reduction by 100% in CFU was observed for SAAP-148-modified polymer in solution against all *E. coli, S. aureus, P. aeruginosa, and A. baumannii* strains.

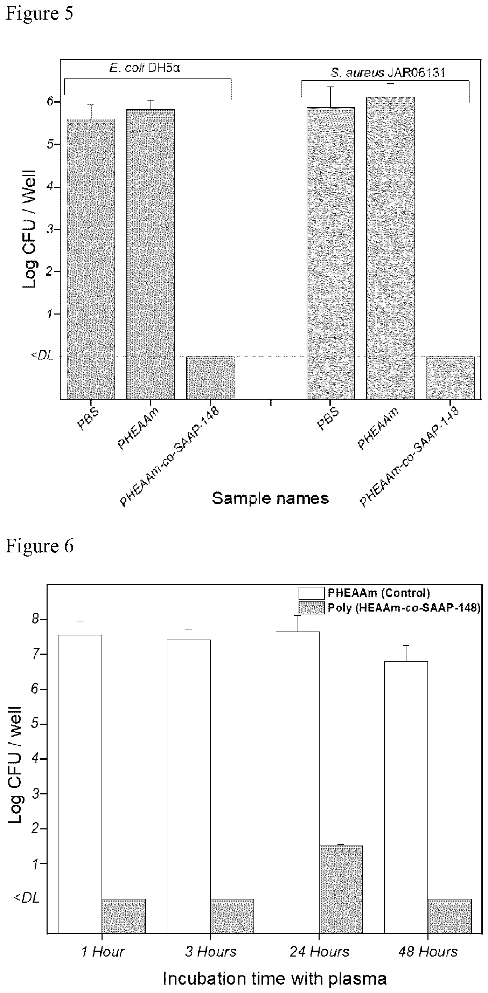

The test was repeated against E. coli DH5α and S. aureus JAR06131 strains with only 2 hours incubation time. Figure 5 illustrates the results of the modified Miles Misra analysis for 2 hours incubation time, where "A" illustrates the result for the PBS control, "B" illustrates the result for the polymer PHEAAm without antimicrobial peptide SAAP-148, and "C" for the peptide-modified poly(HEAAm-*co*-SAAP-148). As can be taken from figure 5, the SAAP-148-modified polymer in solution showed good antimicrobial properties against *E*. *Coli* DH5α and *S*. *aureus* JAR060131 within just 2 hours of incubation time.

These results indicate that the SAAP-148-modified polymer in solution exhibits robust antimicrobial properties and effectively eliminating a diverse range of bacterial strains. This provides promising potential for various biomedical and industrial applications.

### Example 6: Analysis of retention of antimicrobial properties of SAAP-148-modified hydrogels upon exposure to human blood plasma

The retention of the antimicrobial properties of AMP-modified hydrogels was determined in the presence of human blood plasma for *E. coli* as an example.

For this analysis, SAAP-148-modified hydrogel of poly(HEAAm-*co*-SAAP-148) was immobilized in a 96-well plate, as described above. To each well, 100 µL of 20% human plasma diluted in sterile Milli-Q water was added and samples were incubated at 37 °C from 1 hour to 48 hours. The hydrogel was washed with PBS following the extraction of the liquid medium at each time point. *E. coli* DH5α suspension (100 µL of 10⁶ CFU/mL) in RPMI was added and incubated overnight at 37 °C. Afterwards, 10 µL aliquots of each suspension were cultured in a dilution series onto the blood agar plates with overnight incubation, and then the number of colonies were counted to investigate how long the SAAP-148-modified hydrogels retain their bactericidal activity in the presence of human blood plasma. SAAP-148-free hydrogel poly(HEAAm) (PHEAAm) was used as a control.

Figure 6 illustrates the results for the antimicrobial activity against E. coli DH5α for SAAP-148-modified hydrogels in contact with human blood plasma. As can be taken from figure 6, the SAAP-148-modified hydrogel demonstrated its antimicrobial effect in human blood plasma against *E. coli* DH5α for up to 48 hours, showing a significant reduction in log CFU (only 1 to 30 CFU/mL remaining), as compared to a hydrogel without SAAP-148 as a control sample, where a considerable number of bacterial colonies (10^{6.3} to 10^{6.8} CFU/mL) was observed.

The results suggest that the covalent attachment of SAAP-148 can considerably increase the lifetime of peptide functionality compared to when the peptides are solubilized.

### Example 7: Cytotoxicity testing of poly(HEAAm-co-SAAP-148)

The viability of human lung fibroblasts exposed to solutions of (a) SAAP-148-modified polymer poly(HEAAm-co-SAAP-148) and (b) SAAP-148-free polymer poly(HEAAm) (PHEAAm) with a series of concentrations was studied with the WST-1 assay. This method is based on the conversion of the tetrazolium salt WST-1 to formazan by mitochondrial dehydrogenases. As the number of viable cells increases, their active mitochondria produce more dehydrogenase enzymes for the WST-1 reaction. The amount of formazan produced directly correlates with the number of viable cells and can be quantified by measuring the absorbance of the dye at a specific wavelength, typically 440 nm.

For this experiment, 0.1 - 5 × 10⁴ normal human lung fibroblast (NHLF) cells per well were cultured in a 96-well microtiter plate with a final volume of 100 µL of culture medium. The cells were incubated for 24 hours, after which the old media was removed and replaced with 100 µL of new media containing Dulbecco's Modified Eagle's Medium (DMEM) and 2% fetal bovine serum (FBS). Series dilutions of 100 µL solution of SAAP-148-free and SAAP-148-modified polymers with concentration 25mg/mL (2 replicates each) were added to each well separately and incubated for an additional 24 hours. Afterwards, 10 µL of WST-1 reagent was added to each well and incubated for 30 minutes. The colour of the media changed from pink to yellowish after 30 minutes. The reaction was then stopped by adding 10 µL of 1% sodium dodecyl sulfate (SDS) to each well and shaking the plate for 1 minute to mix the contents. Absorbance of the control (untreated) and treated samples was measured using a microplate reader at the wavelength of 400 nm. 100 µL culture medium with 10 µL WST-1 was used as blank control in separate wells.

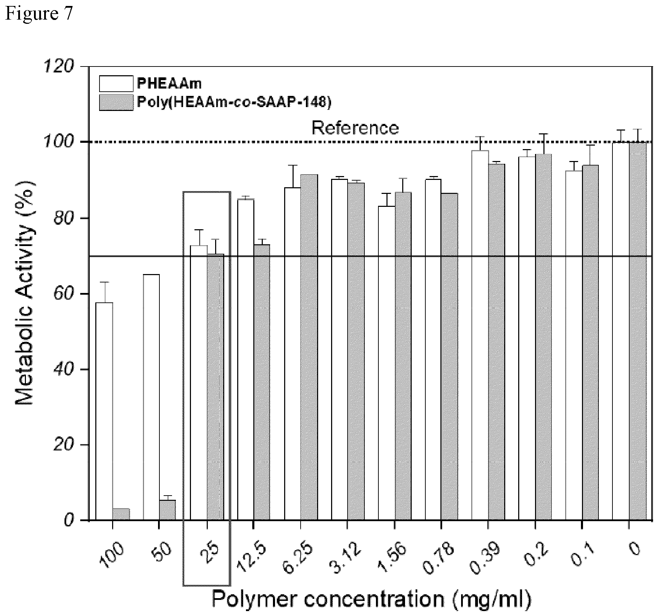

Figure 7 shows the results of the assessment of metabolic activity by the WST-1 assay for NHLF cells in contact with solutions of single chain polymers without (PHEAAm) and with covalently attached antimicrobial peptides (poly(HEAAm-co-SAAP-148)). The results as shown in Figure 7 illustrated that more than 70% of the cells maintained metabolic activity when in contact with the SAAP-148-modified polymer solution at a concentration of 25 mg/mL and below. This indicates low toxicity according to the international standard test ISO 10993-5:2009 for medical devices.

In summary, these results show that an antimicrobial copolymer comprising a hydrophilic monomer, a monomer functionalized with a photocrosslinkable group and a monomer functionalized with an antimicrobial peptide that is covalently bound via at least one of its amino groups was synthesized by covalently immobilization of the antimicrobial peptide (AMP) SAAP-148 at the active ester sites of the parent copolymer poly(HEAAm-co-BPAAm-co-PFPA-co-EH3A). The polymer was not toxic to human lung fibroblasts and was further transformed into a photocrosslinked hydrogel by irradiation with UV light and water swelling. This material showed a broad spectrum of antimicrobial activity against both Gram-positive and Gram-negative bacterial strains. The covalently immobilized antimicrobial peptide (AMP) SAAP-148 did not leach out to the surrounding medium and the hydrogel retained its antimicrobial functionality in 20% human blood plasma for up to 48 hours.

### Example 8:

### Synthesis of a copolymer functionalized with a chromogenic dye convertible by a bacterial enzyme

Acrylate-based monomers were synthesized according to examples 1.1 to 1.3.

In step 1, poly(HEAAm-*co*-BPAAm-*co*-PFPA-*co*-EH3A) was synthesized. In a next step 2, this copolymer was functionalized with N-tert.-butyloxycarbonyloxy (Boc)-protected hexamethylene diamine (Boc-HDMA) via active easter chemistry yielding poly(HEAAm-*co*-BPAAm-*co*-boc-HMDA-*co*-EH3A). This synthesized copolymer was deprotected in step 3 to obtain free amine in the polymer backbone (poly(HEAAm-*co*-BPAAm-*co*-HMDA-*co*-EH3A)). In step 4, two chromogenic dyes (X-GLUC and PNPG) were modified with CDI using active ester chemistry to enable their use in the subsequent step. The obtained copolymer in step 3 was modified with functional dyes (synthesized in step 4) to form poly[(hydroxy ethyl acrylamide)-*co*-(4-benzophenone acrylamide)-*co*-(5-bromo-4-chloro-3-indolyl β-D-glucuronide)-*co*-(ECOSURF EH3 acrylate)] and poly[(hydroxy ethyl acrylamide)-*co*-(4-benzophenone acrylamide)-*co*-(4-nitrophenyl-β-D-glucopyranosiduronic acid)-*co-*(ECOSURF EH3 acrylate)] in step 5a and 5b. The copolymers were confirmed by ¹H NMR.

### Step 1:

To synthesize the polymer poly[(hydroxy ethyl acrylamide)-co-(4-benzophenone acrylamide)-co-(pentafluorophenyl acrylate)-co-(ECOSURF EH-3 acrylate)] (poly(HEAAm-*co*-BPAAm-*co*-PFPA-*co*-EH3A)), 500 mg (4.41 mmol) of HEAAm, 72.60 mg (0.13 mmol) of EH3A, 22.20 mg (0.08 mmol) of BPAAm, 73.60 mg (0.30 mmol) of PFPA, and 7.25 mg (0.44 mmol, 1 mol%) of AIBN were combined in a Schlenk tube. 10 mL of methanol was added, and the mixture was purged with argon before heating at 65°C for 24 hours. The resulting polymer was precipitated in diethyl ether, filtered, and dried under vacuum, yielding the product.

### Step 2:

250 mg (1.05 mmol) of the synthesized polymer poly(HEAAm-co-BPAAm-co-PFPA-co-EH3A), 159 mg (0.73 mmol, 10 eq.) of Boc-HDMA, and 7.4 µL of TEA were combined in a Schlenk tube with 10 mL of methanol: chloroform (8:1) solvent. The mixture was stirred at room temperature for 120 hours. The product was precipitated in acetone, filtered, dried, redissolved in water, dialyzed, and finally freeze-dried, yielding the product (poly(HEAAm-*co*-BPAAm-*co*-Boc-HMDAAm-*co*-EH3A)).

### Step 3:

100 mg of the Boc-protected polymer poly(HEAAm-*co*-BPAAm-*co*-Boc-HMDAAm-*co*-EH3A) was dissolved in 1.5 mL of methanol, and 1.5 mL of TFA was added to deprotect the amine groups. The reaction mixture was stirred overnight, precipitated in ice-cold acetone, filtered, and redissolved in water. The solution was then freeze-dried to obtain the amino end-functionalized polymer. Subsequently, the polymer was dissolved in 5 mL of water, Lewatit MP62 was added for anion exchange, and the mixture was stirred overnight. The final product poly(HEAAm-*co*-BPAAm-*co-*HMDAAm-*co*-EH3A) was obtained by filtration, dilution, and freeze-drying.

### Step 4(a):

47 mg (0.14 mmol) of PNPG and 36 mg (0.22 mmol) of Carbonyl diimidazole (CDI) were dissolved in 10 mL of Milli-Q water and stirred at room temperature for 120 hours. The reaction mixture was washed with 10 mL of dichloromethane, the aqueous phase was separated, and the product was obtained by freeze-drying, yielding the product.

### Step 4(b):

150 mg (0.33 mmol) of X-GLUC and 76 mg (0.47 mmol, 1.4 eq.) of CDI were dissolved in 10 mL of Milli-Q water and stirred at room temperature for 120 hours. The reaction mixture was washed with 10 mL of dichloromethane, the aqueous phase was separated, and the product was obtained by freeze-drying, yielding the product.

### Step 5(a):

50 mg (0.0672 mmol) of the synthesized polymer poly(HEAAm-*co*-BPAAm-*co*-HMDAAm-*co-*EH3A) and 55.108 mg (0.150 mmol, 5 eq.) of PNPG-CDI were dissolved in 10 mL of Milli-Q water, 3 µL of TEA was added, and the mixture was stirred at room temperature for 72 hours. The product was precipitated in ice-cold acetone, redissolved in water, and freeze-dried, yielding the product.

### Step 5(b):

50 mg (0.0672 mmol) of the synthesized polymer poly(HEAAm-*co*-BPAAm-*co*-HMDAAm-*co-*EH3A) and 50.93 mg (0.11 mmol, 5 eq.) of X-GLUC-CDI were dissolved in 10 mL of Milli-Q water, 4 µL of TEA was added, and the mixture was stirred at room temperature for 72 hours. The product was precipitated in ice-cold acetone, redissolved in water, and freeze-dried, yielding the product.

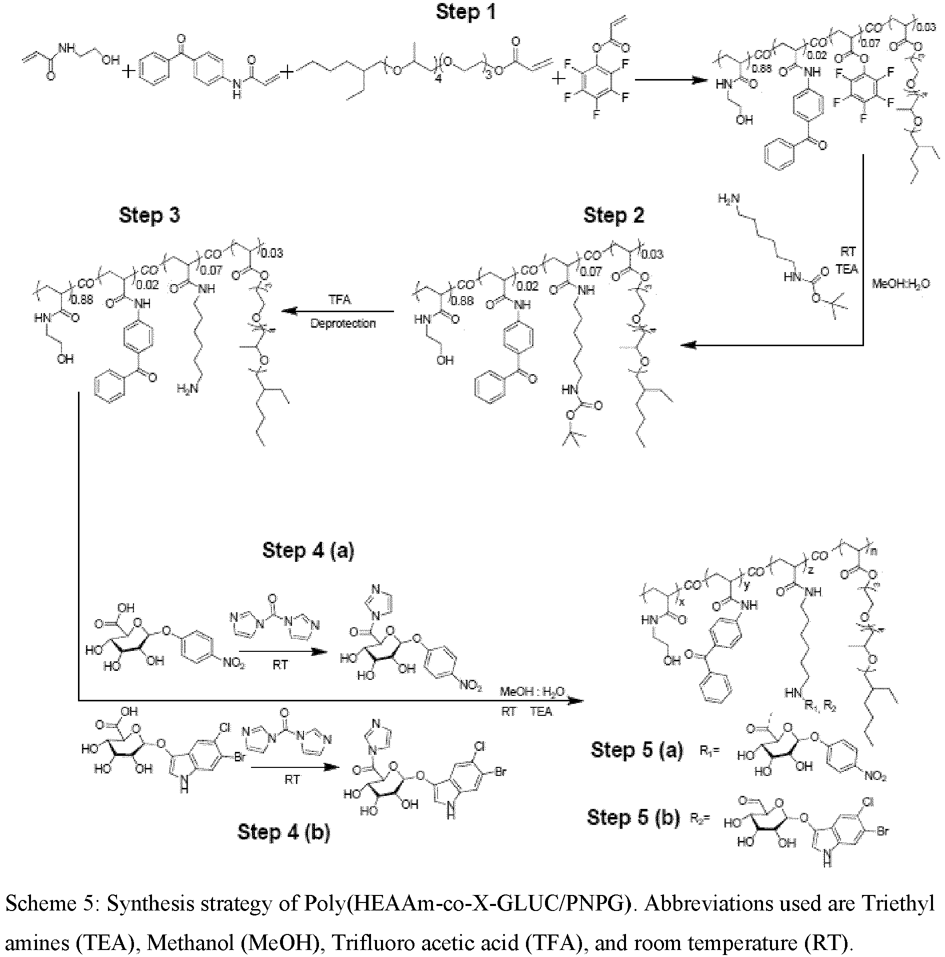

In the following, the copolymer poly[(hydroxy ethyl acrylamide)-*co*-(4-benzophenone acrylamide)-*co-*(5-bromo-4-chloro-3-indolyl-β-D-glucuronide)-*co*-(ECOSURF EH3 acrylate)] is denoted poly(HEAAm-*co*-BPAAm-co-X-GLUC-*co*-EH3A) or in a shortened form as "poly(HEAAm-*co*-X-GLUC)".

Further, the copolymer poly[(hydroxy ethyl acrylamide)-*co*-(4-benzophenone acrylamide)-*co-*(ECOSURF EH3 acrylate)] was used as a control and is denoted poly(HEAAm-co-BPAAm-*co-*EH3A) or in a shortened form as "poly(HEAAm)". These abbreviations also refer to the crosslinked copolymers, where the text differentiates when referring to the crosslinkable or the crosslinked copolymer.

### Example 9: Determination of chromogenic effect of enzyme solution on dye modified polymers in solution and on surface-attached hydrogels

Commercially available β- galactosidase (β-GAS), which was purchased as isolate from *E. coli,* (140 units/mg protein, E.C.3.2.1.23), and β-glucuronidase (β-GUS), isolated from *E. coli* (1000-5000 units/mg protein, E.C.3.2.1.31; Type IX-A) were used as enzymes to determine the chromogenic effect on were used as enzymes to determine the chromogenic effect on dye-modified polymers in solutions and on surface-attached hydrogels.

To study the enzymatic effect with the soluble polymers, 1 millilitre (mL) of dye-modified polymer solutions poly(HEAAm-*co*-BPAAm-*co*-X-GLUC-*co*-EH3A), in the following abbreviated PHXG, and poly(HEAAm-*co*-BPAAm-*co*-PNPG-*co*-EH3A), in the following abbreviated PHPN, were prepared in phosphate-buffered saline (PBS) (pH 7.4) at a concentration of 25 milligrams per millilitre (mg/mL) and subsequently added to separate glass vials. 10 microliters (µL) of freshly prepared β-GAS solution in PBS (pH 7.4) was added to the PHXG polymer solution vial, while 10 microliters (µL) of freshly prepared β-GUS solution in PBS (pH 7.4) was added to the PHPN polymer solution vial, both at a final enzyme concentration of 0.1 micromolar (µM). Visual changes of the solution color were then monitored.

It was detected by naked eye that the PHXG polymer solution assumed an indigo color. This effect was attributed to the β-linked indole derivative of X-GLUC chromogenic substance being cleaved by β-GAS purified from *E. coli* (E. C. 3.2.1.23,4-methylumbelliferyl-b-D-glucuronide hydrate) and its release into the polymer solution, where the liberated molecule dimerizes to form a derivative, which is water insoluble and possesses a distinctive indigo color.

It was further detected by naked eye that the PHPN polymer solution assumed a yellow color. This effect was attributed to the polymer containing PNPG being cleaved by β-GUS purified from *E. coli* (1000 000-5000000 units/g protein, E.C.3.2.1.31; Type IX-A) and liberating 4-nitrophenol (4-NP) from the glucuronide, whose yellow color, which is due to the deprotonation of 4-NP, can be observed.

A comparative analysis of the chromogenic effect was conducted between dye-modified polymers in solution (PHXG and PHPN) containing chromogenic substances, and a reference polymer poly(HEAAm-*co*-BPAAm-*co*-EH3A) without chromogenic substances in aqueous solution as control (PHEAAm). It was observed that the control solution did not undergo a color change following enzyme addition. In contrast, indigo and yellow color development observed in solutions of PHXG and PHPN, respectively, indicates that the chromogenic substances remain active when covalently immobilized within the polymer backbone and cleavage occurs during the enzymatic reactions with β-GAS and β-GUS respectively.

The chromogenic effect was also examined in hydrogels made from PHXG and PHPN. Dye-modified polymer solutions of 25 mg/mL PHXG and PHPN in methanol and water (90:10, v/v, pH 7.4) were poured into separate petri dishes, dried on a heating plate and then photocrosslinked under UV light (wavelength 365 nm) to form polymer networks. These networks were subsequently swollen in water to form hydrogels. Fresh enzyme solutions (10 µL of β-GAS and β-GUS, 0.1 µM concentration in PBS, pH 7.4) were prepared. β-GAS was added to PHXG hydrogels while β-GUS was added to PHPN hydrogels and color changes were observed visually. Similar to the dye-modified polymer solutions, the chromogenic substances within the hydrogels also produced indigo and yellow colors respectively upon exposure to the enzyme solutions. This confirms that the chromogenic substances remain active even after being immobilized within the hydrogel matrix and were accessible by the enzymes via diffusion through the hydrogel network.

### Example 10: Detection of bacterial strains by dye-modified single chain polymers in solutions

Clinical and laboratory strains of *Escherichia coli* (PC 1568, DH5α, DC 10B, B12 C1, TOP 10, PC 2348AR4, ATCC 8739, and ML35 (ATCC 43827), *Staphylococcus aureus* strain JAR060131, a standard laboratory strain of *Pseudomonas aeruginosa* PAO1, and a multidrug-resistant strain of *Acinetobacter baumannii* RUH875 from frozen stocks were grown at 37 °C on blood agar plates for 16-18 h. From each plate, overnight cultures were prepared in lysogeny broth (Oxoid) for all *E. coli* strains, and in tryptic soy broth (Oxoid) for *S*. *aureus, P. aeruginosa,* and *A. baumannii,* and incubated at 37 °C for 18-24 h at 120 rpm. Prior to each experiment, bacteria were cultured to the mid-logarithmic growth phase in their respective media. Bacteria were washed twice with phosphate-buffered saline (PBS; pH=7.0) and diluted in RPMI to 1 × 10⁶ colony forming units (CFU)/mL based on the optical density at 620 nm.

100 µL of polymer solutions (PHXG and PHPN) were added to each well of 96 well plates following the addition of 100 µL (10⁶ CFU/mL) bacterial suspension and plates were incubated for overnight at 37 °C. Afterwords the color changes were observed visually by naked eyes.

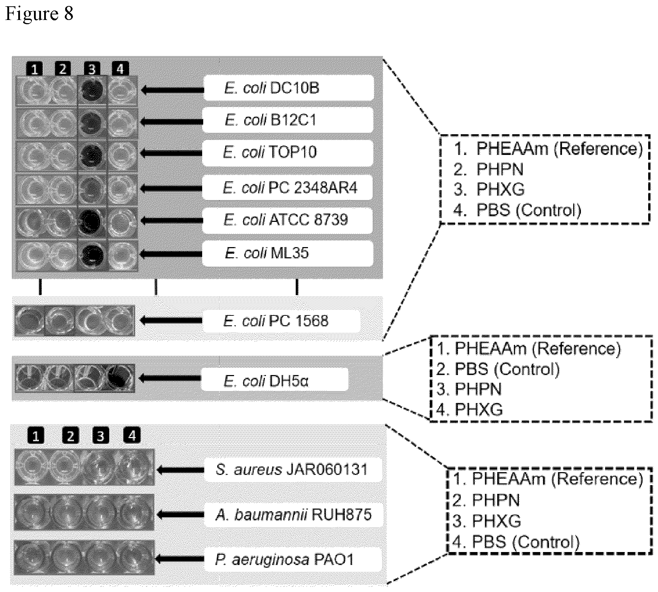

Figure 8 shows the color changes of copolymers poly(HEAAm-*co*-PNPG) and poly(HEAAm-*co*-X-GLUC) by enzymatic reaction of different strains of E. coli, S. aureus, P. aeruginosa, and A. baumannii. The results showed that when treating PHXG polymer solutions with suspensions of *E. coli* strains DC 10B, B12 C1, TOP10, PC 2348AR4, ATCC 8739, and ML35 an indigo color change was observed. The results indicate the production of galactosidase enzyme by these strains in sufficient quantities to induce the cleavage of the chromogenic dyes. Conversely, no color change was observed when exposing a PHPN solution to these bacterial strains, suggesting either the absence or insufficient production of glucuronidase by these strains. *E. coli* strain PC 1568 elicited a yellow color change in PHPN solution, confirming glucuronidase activity. Strain DH5α induced color changes in both PHXG and PHPN, indicating the production of both galactosidase and glucuronidase enzymes. No color alterations occurred in PHXG and PHPN solutions when exposed to *S*. *aureus* (JAR060131), *P. aeruginosa* (PA01), and *A. baumannii* (RUH875), suggesting either the lack of glucuronidase and galactosidase production or insufficient enzyme secretion for detectable color changes in the assay. These findings demonstrate that all tested *E. coli* strains were detectable using the copolymers functionalized with chromogenic dye.

### Example 11: Cytotoxicity testing of poly(HEAAm-co-X-GLUC) and poly(HEAAm-co-PNPG)

The viability of human lung fibroblast exposed to solutions of dye-modified polymers PHXG and PHPN, as well as the reference polymer PHEAAm without dye modification was studied with a series of polymer concentrations employing the WST-01 assay.

For this experiment, 0.1-5×10⁴ NHLF cells per well were cultured in a 96-well microtiter plate with a final volume of 100 µL of DMEM with 2% FBS. The cells were incubated for 24 h, after which the medium was replaced with 100 µL of fresh DMEM with 2% FBS. Two-fold serial dilutions of pure polymer (without dye modification) and dye-modified polymer solutions with a concentration 25 mg/mL (2 replicates each; final volume of 100 µL) were added to separate wells and incubated for an additional 24 h. Subsequently, 10 µL of WST-1 reagent was added to each well and incubated for 30 min. The colour of the media changed from pink to yellowish after 30 min. The reaction was then stopped by adding 10 µL of 1% SDS to each well and shaking the plate for 1 min to mix the contents. Absorbance of the (untreated) control and treated samples was measured using a microplate reader (Synergy H1, Biotek, USA) at a wavelength of 400 nm. One hundred µL culture medium with 10 µL WST-1 was used as blank control in separate wells.

The results demonstrated that over 70% of the cells maintained metabolic activity when in contact with dye-modified polymer solutions at a concentration of 25mg/mL and below after exposure to the dye-modified polymer solutions. This finding suggests low toxicity according to the international standard ISO 10993-5:2009 test for medical devices

The work leading to this invention has received funding from the European Union's Horizon 2020 research and innovation programme under the Marie SKLODOWSKA-CURIE grant agreement No 955664.

## Claims

1. A crosslinkable copolymer, the crosslinkable copolymer comprising a hydrophilic monomer, a monomer functionalized with a photocrosslinkable group and a monomer functionalized with an antimicrobial peptide that is covalently bound via at least one of its amino groups.

2. The crosslinkable copolymer according to claim 1, wherein the crosslinkable copolymer comprises a monomer functionalized with a chromogenic dye convertible by a bacterial enzyme, wherein the chromogenic dye optionally is selected from the group comprising 4-nitrophenyl β-D-glucuronide, 5-bromo-4-chloro-3-indolyl-β-D-glucuronide, 4-methylumbelliferyl β-D-glucuronide and 4-methylumbelliferyl α-D-glucuronide, and wherein the monomer optionally is N-(6-aminohexyl)acrylamide.

3. The crosslinkable copolymer according to claim 1 or 2, wherein the crosslinkable copolymer comprises a monomer functionalized with a surfactant, wherein the surfactant optionally is selected from the group comprising ethylene oxide-propylene oxide copolymer mono(2-ethylhexyl) ethers, poly(oxyethylene) lauryl ethers and 4-(1,1,3,3-tetramethylbutyl)phenyl-polyethylene glycols, and wherein the monomer optionally is selected from the group comprising acrylate and methacrylate.

4. The crosslinkable copolymer according to any one of the preceding claims, wherein the antimicrobial peptide is selected from the group of peptides comprising acetyl-LKRVWKRVFKLLKRYWRQLKKPVR (SAAP-148) (SEQ ID NO: 1), acetyl-LKRLYKRLAKLIKRLYRYLKKPVR-amide (SAAP-145) (SEQ ID NO: 2), acetyl-LKRVWKAVFKLLKRYWRQLKKPVR-amide (SAAP-276) (SEQ ID NO: 3), acetyl-IGKEFKRIVERIKRFLRELVRPLR-amide (OP-145) (SEQ ID NO: 4), LRCMCIKWWSGKHPK-amide (TC19) (SEQ ID NO: 5), LRAMCIKWWSGKHPK-amide (TC84) (SEQ ID NO: 6) and GKWKLFKKAFKKFLKILAC (BP2) (SEQ ID NO: 7).

5. The crosslinkable copolymer according to any one of the preceding claims, wherein the hydrophilic monomer is selected from the group of monomers comprising hydroxyethyl acrylamide, hydroxypropyl acrylamide, N-vinyl-2-pyrrolidone, N,N-dimethylacrylamide, methacrylamide, 2-hydroxyethyl methacrylate, 2-hydroxypropyl methacrylate, 2-methyl-2-oxazoline, 2-ethyl-2-oxazoline, 2-n-propyl-2-oxazoline and 2-isopropyl-2-oxazoline.

6. The crosslinkable copolymer according to any one of the preceding claims, wherein the monomer functionalized with a photocrosslinkable group is selected from (4-benzoylphenyl)acrylamide and 4-benzoylphenyl)methacrylamide, and optionally is N-(4-benzoylphenyl)acrylamide.

7. The crosslinkable copolymer according to any one of the preceding claims, wherein the antimicrobial peptide binds to an acrylate monomer functionalized with pentafluorophenole via reaction of at least one of its amino group(s) forming an amide bond with the acrylate.

8. The crosslinkable copolymer according to any one of the preceding claims, wherein the crosslinkable polymer has the following formula (1): 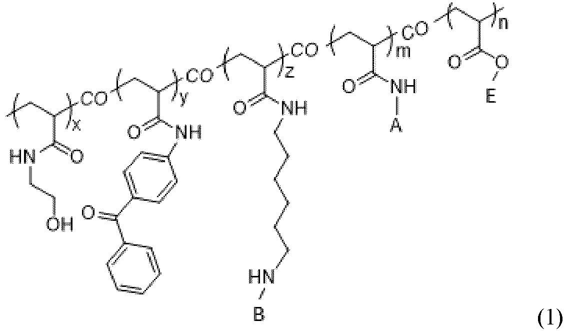 wherein:
A is an antimicrobial peptide selected from the group of SAAP-148 (SEQ ID NO: 1), SAAP-145 (SEQ ID NO: 2), SAAP-276 (SEQ ID NO: 3), OP-145 (SEQ ID NO: 4), TC19 (SEQ ID NO: 5), TC84 (SEQ ID NO: 6) and BP2 (SEQ ID NO: 7),
B is a chromogenic dye selected from the group comprising 4-nitrophenyl β-D-glucuronide (PNPG), 5-bromo-4-chloro-3-indolyl-β-D-glucuronide (X-Gluc), 4-methylumbelliferyl β-D-glucuronide (MUG) and 4-methylumbelliferyl α-D-glucuronide (MUD),
E is a surfactant selected from the group of ethylene oxide-propylene oxide copolymer mono(2-ethylhexyl) ethers, poly(oxyethylene) lauryl ethers and 4-(1,1,3,3-tetramethylbutyl)phenyl-polyethylene glycols,
x is in a range of 0.6 to 0.95, preferably 0.88,
y is in a range of 0.01 to 0.03, preferably 0.02,
z is in a range of 0 to 0.10, preferably 0.01 to 0.10, more preferably 0.07,
m is in a range of 0.0001 to 0.1, preferably 0.001,
n is in a range of 0 to 0.03, preferably 0.01 to 0.03, more preferably 0.02.

9. The crosslinkable copolymer according to any one of the preceding claims, wherein the crosslinkable polymer is selected from the group of the following polymers: 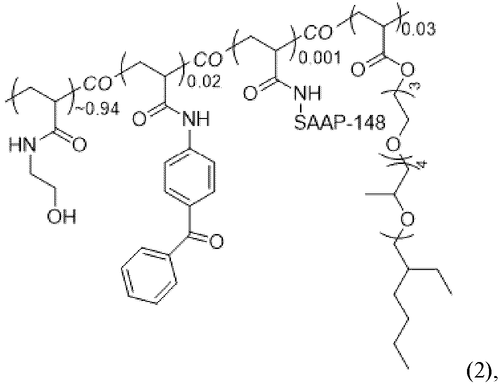 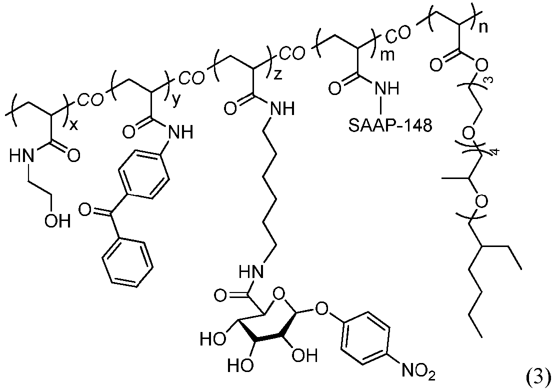 wherein
x is in a range of 0.6 to 0.95, preferably 0.88,
y is in a range of 0.01 to 0.03, preferably 0.02,
z is in a range of 0.01 to 0.10, preferably 0.07,
m is in a range of 0.0001 to 0.1, preferably 0.001, and
n is in a range of 0.01 to 0.03, preferably 0.02, 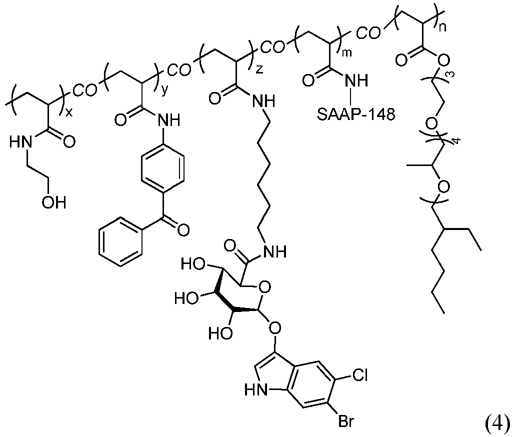
wherein
x is in a range of 0.6 to 0.95, preferably 0.88,
y is in a range of 0.01 to 0.03, preferably 0.02,
z is in a range of 0.01 to 0.10, preferably 0.07,
m is in a range of 0.0001 to 0.1, preferably 0.001,
n is in a range of 0.01 to 0.03, preferably 0.02.
and

10. A crosslinked copolymer, obtained by photo-induced crosslinking of one or more of the crosslinkable polymer(s) according to any one of claims 1 to 9.

11. A hydrogel, comprising the crosslinked copolymer according to claim 10.

12. A wound dressing, comprising one or more of the crosslinkable polymer(s) according to any one of claims 1 to 9, the crosslinked copolymer according to claim 10 or the hydrogel according to claim 11.

13. The hydrogel according to claim 11 or the wound dressing according to claim 12 for use in the diagnosis and/or treatment of a bacterial infection.

14. The hydrogel or the wound dressing for use according to claim 13, wherein the bacterial infection is caused by *Escherichia coli (E. coli)*, *Staphylococcus aureus*, *Pseudomonas aeruginosa (P. aeruginosa) and*/*or Acinetobacter baumanii (A. baumanii).*
